(19) **Europäisches Patentamt**
**European Patent Office**
**Office européen des brevets**

(11) **EP 3 368 553 B1**

(12) **EUROPEAN PATENT SPECIFICATION**

(45) Date of publication and mention
of the grant of the patent:
**02.08.2023 Bulletin 2023/31**

(21) Application number: **16860747.1**

(22) Date of filing: **27.10.2016**

(51) International Patent Classification (IPC):
**A61K 31/137** (2006.01)    **C07K 14/47** (2006.01)
**A61K 31/426** (2006.01)    **A61K 31/4439** (2006.01)
**A61K 47/58** (2017.01)    **A61K 47/59** (2017.01)
**A61P 5/48** (2006.01)

(52) Cooperative Patent Classification (CPC):
**A61K 31/137; A61K 31/426; A61K 31/4439;**
**A61K 47/593; A61K 47/605; A61P 5/48;**
**C07K 14/47**

(86) International application number:
**PCT/US2016/058997**

(87) International publication number:
**WO 2017/075136 (04.05.2017 Gazette 2017/18)**

(54) **POLYMER-BASED THERAPEUTICS FOR INDUCTIVE BROWNING OF FAT**

THERAPEUTIKA AUF POLYMERBASIS FÜR INDUKTIVE BRÄUNUNG VON FETT

PRODUITS THÉRAPEUTIQUES À BASE DE POLYMÈRE POUR LE BRUNISSEMENT INDUCTIF
DE GRAISSE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **27.10.2015 US 201562247192 P**

(43) Date of publication of application:
**05.09.2018 Bulletin 2018/36**

(73) Proprietor: **Purdue Research Foundation**
**West Lafayette, IN 47906 (US)**

(72) Inventors:
 • **DENG, Meng**
 **West Lafayette, Indiana 47906 (US)**
 • **JIANG, Chunhui**
 **Plano, Texas 75093 (US)**
 • **KUANG, Liangju**
 **West Lafayette, Indiana 47906 (US)**
 • **KUANG, Shihuan**
 **West Lafayette, Indiana 47906 (US)**

(74) Representative: **Zwicker, Jörk**
**ZSP Patentanwälte PartG mbB**
**Hansastraße 32**
**80686 München (DE)**

(56) References cited:
**EP-A1- 2 085 120**         **WO-A1-2015/120065**
**WO-A2-2015/123576**   **WO-A2-2015/123576**
**US-A1- 2014 057 837**   **US-A1- 2015 010 630**

• **PENGPENG BI ET AL: "Inhibition of Notch**
**signaling promotes browning of white adipose**
**tissue and ameliorates obesity", NATURE**
**MEDICINE, vol. 20, no. 8, 1 August 2014**
**(2014-08-01) , pages 911-918, XP055580950, New**
**York ISSN: 1078-8956, DOI: 10.1038/nm.3615**
• **BI et al.: "Inhibition of Notch signaling promotes**
**browning of white adipose tissue and ameliorates**
**obesity", Nat Med., vol. 20, no. 8, August 2014**
**(2014-08), pages 911-918, XP055580950, DOI:**
**doi:10.1038/nm.3615**
• **CYPESS et al.: "Brown fat as a therapy for obesity**
**and diabetes", Curr Opin Endocrinol Diabetes**
**Obes, vol. 17, no. 2, April 2010 (2010-04), pages**
**143-149, XP002780824, DOI:**
**doi:10.1097/MED.0b013e328337a81f**
• **TEASDALE et al.: "Polyphosphazenes:**
**Multifunctional, Biodegradable Vehicles for Drug**
**and Gene Delivery", Polymers, vol. 5, no. 1, 8**
**February 2013 (2013-02-08), pages 161-187,**
**XP055596564,**

Remarks:

The complete document including Reference Table(s) and the Sequence Listing(s) can be downloaded from the EPO website

**Description**

This application claims the benefits under 35 U.S.C. 119 (e) US Provisional Application No. 62/247,192, filed on October 27, 2015.

## TECHNICAL FIELD

The present disclosure generally relates to treatment of obesity and diabetes, and in particular to novel polymer-based systems that allow controlled generation of brown/beige adipose tissues.

## BACKGROUND

This section introduces aspects that may help facilitate a better understanding of the disclosure. Accordingly, these statements are to be read in this light and are not to be understood as admissions about what is or is not prior art.

The obesity epidemic has posed a major concern for public health in modern society due to its association with a spectrum of metabolic diseases including type 2 diabetes (T2D), heart diseases, hyperglycemia and multiple cancers. Obesity is morphologically characterized by the excessive lipid storage in the white adipose tissue (WAT) and featured as a complex disorder of energy imbalance wherein intake outpaces expenditure. Considerable efforts have been devoted to explore the pathological mechanisms as well as the cellular and molecular therapeutic targets to combat obesity.

Currently, there are few medications available for obesity treatment. Mostly, those therapies are dedicated to decreasing energy intake by either suppressing appetite through stimulating the central nervous system or reducing nutrient digestion and absorption within the gastrointestinal tract. However, those medications only produce modest effects and are usually accompanied with unpleasant, potentially harmful side effects. Thus, alternative approaches to overcoming obesity are under extensive exploration. One such approach is through boosting energy expenditure. Recently, this strategy through stimulating thermogenesis has been emerging as an appealing alternative.

Brown adipose tissue (BAT) is the primary site of thermogenesis through uncoupling mitochondrial respiration and ATP synthesis via uncoupling protein 1 (Ucp1). While producing heat, BAT consumes not only free fatty acid but also large amounts of glucose, thus providing benefits to metabolic health. Classical BAT is abundant in neonates but diminishes with age leading to limited quantities in adult humans. This compromises the therapeutic value of BAT in clinical intervention of obesity.

The recent discovery of inducible BAT (iBAT), also called beige adipose tissue, in WAT depots reignites the promise of obesity treatment through stimulating energy expenditure. Beige adipocytes also possess robust thermogenic capacity.

WO2015123576 discloses a nanoparticle comprising a PPAR gamma activator for inducing the browning of white adipose tissue for the treatment of obesity and diabetes, wherein the nanoparticle can be PLGA and/or phosphazene-based. US2014057837 discloses a sustained release administration by encapsulation with PLGA polymers of an agonist, namely a beta adrenergic agonist, in a method for increasing the brown adipose tissue for the treatment of obesity. In addition, in EP2085120 the use of a peptide in combination with a gamma secretase inhibitor for the treatment of obesity has been disclosed. Further US2015010630 discloses compositions for use in the treatment of obesity, comprising silk fibroin-based matrix comprising macrophage-skewing agents, wherein the agents can be encapsulated and wherein the agent can be a Notch signaling inhibitor. Also, in WO2015120065, the administration of a secretase inhibitor inside a bio-capsule for use in the treatment of obesity has been disclosed.

A study (PENGPENG BI, et al., "Inhibition of Notch signaling promotes browning of white adipose tissue and ameliorates obesity", NATURE MEDICINE, vol. 20, no. 8, 1 August 2014 (2014-08-01), pages 911-918) has further disclosed that Notch inhibition induces Ppargcla and Prdm16 transcription in white adipocytes. The pharmacological inhibition of Notch signaling in obese mice has been shown to ameliorate obesity, reduce blood glucose and increase Ucpl expression in white fat. Therefore, Notch signaling may be therapeutically targeted to treat obesity and type 2 diabetes. The same study reveals that said mice may be treated by intraperitoneal injection of the disclosed pharmacological inhibitor.

Current strategies for enhancement of beige adipocyte biogenesis and function involve genetic and systemic pharmacological manipulations, which are associated with significant translational challenges due to the risks of having unintended adverse consequences on other cells/tissues, and lack of control over the location and temporal extent of beige adipocyte biogenesis. Thus, there is a need to provide an effective polymer-based drug delivery system that induces beige adipocyte biogenesis in a spatio-temporally controlled manner, and methods for making such systems.

The references to methods of treatment in the summary and detailed description of the invention in this description are to be interpreted as references to the compounds, pharmaceutical compositions and medicaments of the present invention for use in a method for treatment of the human (or animal) body by therapy (or for diagnosis)."

## SUMMARY

This disclosure presents a therapeutic agent for use to selectively induce browning of adipocytes in a subject. The therapeutic agent comprises at least one compound or composition that induces the formation of brown and/or beige adipocytes; and a polymer that encapsulates the at least one compound or composition, wherein the compound or composition is encapsulated within the polymer via physical interactions or chemical interactions and configured to the proximity to white adipose tissue (WAT).

In one preferred embodiment, the aforementioned therapeutic agent upregulates uncoupling protein-1 (UCP1) expression.

In one preferred the embodiment, the aforementioned therapeutic agent comprises at least one compound or composition that is an inhibitor to Notch signaling pathway.

In some embodiment, the inhibitor to Notch signaling pathway upregulates the expression of *Ucpl, Ppargc1a.*

In one embodiment, the inhibitor to Notch signaling pathway is selected from γ-secretase inhibitors including dibenzazepine (DBZ) and (N-[N-(3,5-difluorophenacetyl)-1-alanyl]-S-phenylglycine t-butyl ester) (DAPT).

In one preferred the embodiment, the aforementioned therapeutic agent comprises the polymer that forms microparticles or nanoparticles.

In one preferred the embodiment, the aforementioned therapeutic agent comprises a polymer selected from the group consisting of synthetic polymers and natural polymers.

In one preferred the embodiment, the aforementioned therapeutic agent comprises a polymer selected from the group consisting of biodegradable polymers and non-degradable polymers.

In one preferred the embodiment, the aforementioned therapeutic agent comprises a polymer comprising a class of artificial vesicles made from synthetic amphiphilic block copolymers, wherein the artificial vesicles comprise hollow spheres that contain an aqueous solution in the core surrounded by a bi-layer membrane.

In one preferred the embodiment, the aforementioned therapeutic agent comprises a polymer of poly (lactic-co-glycolic acid) (PLGA).

In one preferred the embodiment, the aforementioned therapeutic agent is a biodegradable polyphosphazene-γ-secretase inhibitor conjugate.

In one preferred the embodiment, the aforementioned therapeutic agent comprises at least one compound or composition that is selected from β-adrenergic activators (e.g. CL316, 243 and mirabegron), catecholamines (e.g. norepinephrine), PPAR-γ agonists (e.g. rosiglitazone and thiazolidinediones), Capsinoids and capsinoid-like compounds (e.g. grains of paradise extracts), adipose inflammation related cytokines (e.g. IL4), natriuretic peptides (e.g. atrial natriuretic peptide (ANP), brain-type natriuretic peptide (BNP) and C-type natriuretic peptide (CNP)), FGF family members (e.g. FGF1, 15/19,21), BMP family members (e.g. BMP7, BMP8b), thyroid hormones (e.g. T3 and T4 thyroid hormones) and their receptor agonists (e.g. T3 receptor agonists), myokines (e.g. Irisin and meteorinlike (METRNL), VEGF family members (e.g. VEGF-A), Notch inhibitors (e.g. γ-secretase inhibitors), Janus kinase inhibitors (e.g. tofacitinib), spleen tyrosine kinase inhibitors (e.g. R406), prostaglandins and their synthetic analogs, cyclooxygenase-2 agonists, retinoic acid and retinaldehyde, retinaldehyde dehydrogenases activators, adenosine and its receptor activators, parathyroid hormone-related protein (PTHrP), adipokine (e.g. neuregulin 4, adiponectin), orexin, and microRNAs/siRNAs targeting the aforementioned factors related signaling pathways, or the combination thereof.

In one preferred the embodiment, the aforementioned therapeutic agent further comprises adipocytes, adipocyte progenitor cells, or stem cells embedded in the polymer.

This disclosure further provides a method of delivering therapeutic amount of a drug to induce browning of adipocytes in a subject. The method comprises encapsulating a drug to a polymer matrix system, and releasing the drug at the proximity of WAT, wherein the drug is a compound or composition that induces the formation of brown and/or beige adipocytes.

This disclosure further provides a method of treating obesity in a subject. The method comprises using the aforementioned therapeutic agent.

In one embodiment, the aforementioned method of delivering therapeutic amount of a drug further comprising seeding adipocytes, adipocyte progenitor cells, and stem cells onto the polymer matrix system to engineer brown/beige adipose tissues using scaffold-based tissue engineering principles.

In one embodiment, the encapsulation of the drug to the polymer matrix system is developed by physical interactions selected from any one of or a combination of hydrophobic interaction, hydrophilic interaction, hydrogen bonding, and inter-molecular electrostatic interactions.

In one embodiment, the encapsulation of the drug to the polymer matrix system is developed through a conjugation linkage between the drug and the polymer via at least one functional group.

In one embodiment, the polymer-drug conjugation linkage is formed by any one of or a combination of amine reaction, thiol reactions (e.g. thiol click reactions), carboxylate reaction, hydroxyl reactions, aldehyde and ketone reactions, active hydrogen reactions, photo-chemical reaction, and cycloaddition reactions (e.g. Diels-alder reaction, copper-catalyzed azide-alkyne cycloaddition (CuAAC), copper-free azide-alkyne huisgen cycloaddition).

In one embodiment, at least one functional group is selected from the group comprises any one of or a combination of amines, thiol, carboxylic acid, aldehyde, ketone, active hydrogen sites on aromatic ring, diene, azide isothiocyanates, isocyanates, acyl azides. N - hydroxysuccinimide (NHS) ester, sulfo-NHS, sulfonyl chloride, aldehydes, epoxides, carbonates, aryl halide, imidoesters, carbodiimides (e.g. N, N'-dicyclohexylcarbodiimide (DCC) and 1-Ethyl-3-(3-dimethylaminopropyl) carbodiimide (EDC)), alkylphosphate compound, anhydride, fluorophenyl ester, hydroxymethyl phosphine, guanidino group, iodoacetyl derivative, maleimides, aziridines, acryloyl derivatives, arylating agents, disulfide derivative, vinylsulfone, phenylthioester, cisplatin, diazoacetate, carbonyl diimidazole, oxiranes, N, N'-disuccinimidyl carbonate (DSC), N-hydroxylsuccinimidyl chloroformate, alkyl halogens, hydrazine, maleimide, alkyne, and phosphorus-bound chlorine.

In one embodiment, the conjugated linkage comprises any one of or a combination of isothiourea, isourea, amide, sulfonamide, shift-base, secondary amine, carbamate, arylamine, amidine, phosphoramidate, thioether, disulfide, β-thiosulfonyl, ester, carbamate, hydrazone, diazo, 2+4 cycloaddition, 1,2,3-triazoles, carbohydrates, amino acid esters bond.

In some embodiment, the polymer matrix are synthetic polymers comprising any one of or a combination of poly (aliphatic ester) (e.g. poly(lactide) (PLA), poly(ε-caprolactone) (PCL), poly(glycolic acid) (PGA), poly(lactic-co-glycolic acid) (PLGA), poly(trimethylene carbonate) (PTMC), polydioxanone (PDS), poly(ortho ester), polyanhydrides, poly(anhydride-co-imide), poly(anhydride-esters), polyurethanes(e.g. Degrapols), poly(glycerol sebacate), poly(ethylene imine), poly(acrylic acid)(PAA), polyethylene glycol (PEG), poly(vinyl alcohol) (PVA), poly(N-isopropylacrylamide) (PNIPAm), poly(oxazolines) (e.g. poly(2-methyloxazoline and poly(2-ethyl-2-oxazoline), oligo(ethylene glycol) fumarates (OPFs), poly(propylene fumarate), poly(alkyl cyanoacrylates), polyacrylic amide, synthetic poly(amino acids) (e.g. poly (L-glutamic acid) (L-PGA) and poly (aspartic acid)), polyphosphazenes, and poly(phosphoesters) and blends thereof.

In some embodiments, the polymer matrix system comprises natural polymers comprising any one of or a combination of fibrin, collagen, matrigel, elastin, elastin-like peptides, albumin, natural poly (amino acids) (e.g. cyanophycin, poly (ε-L-lysine) and poly (x-glutamic acid)), and polysaccharides (e.g. hyaluronic acid, chitosan, dextran, chondroitin sulfate, agarose, alginate, and heparin), and blends thereof.

In some embodiments, the polymer matrix system comprises non-degradable polymers. These non-biodegradable polymers comprise any one of or a combination of poly(ethyl ethylene) (PEE), poly(butadiene) (PBD), poly(dimethylsiloxane) (PDMS), and poly(styrene) (PS), poly (N-ethyl-4-vinypyridinium), poly(2,2-(dimethyl aminoethyl methacrylate), poly(ethylene imine), poly(allylamine), and poly(diallyl dimethyl ammonium chloride), poly(acrylic acid), poly(styrene sulfonate), poly(vinyl sulfate), and poly(3-sulfopropyl methacrylate), and polyacrylic amide and blends thereof.

In some embodiments, the polymer matrix system comprises any one of or a combination of linear, star-shaped, hyper-branched, and crosslinked architectures.

In some embodiments, the polymer-based drug delivery system further comprises one or more surface modifications including, but are not limited to, PEGylation or the use of ligands targeting receptors on adipocytes.

These and other features, aspects and advantages of the present disclosure will become better understood with reference to the following figures, associated descriptions and claims.

## BRIEF DESCRIPTION OF THE FIGURES

Figs. 1A and 1B show a polymer-based drug delivery system that includes a drug encapsulated within a polymer matrix system through (Fig. 1A) physical interactions and (Fig. 1B) chemical interactions.

Fig. 2A is a chart showing how the Notch inhibitor DAPT induces browning of cultured white adipocytes and mRNA levels of Notch targets.

Fig. 2B is a chart showing how the Notch inhibitor DAPT induces browning of cultured white adipocytes and brown adipocyte-related genes.

Fig. 2C is a Western blot showing level of proteins.

Fig. 2D is a graph showing DAPT raised $O_2$ consumption rate (OCR). $*P < 0.05$, $**P < 0.01$.

Fig. 3A is a picture of the dorsal view of the mice showing inhibition of Notch in *Leptin* deficient obese mice ($Lep^{ob}$) ameliorates obesity and induces browning.

Fig. 3B is an image of subcutaneous WAT and H&E staining (right, scale bar 100 μm).

Fig. 3C shows plasma glucose measurements.

Fig. 3D is a graph showing relative expression of adipocyte genes in subcutaneous WAT.

Fig. 3E shows UCP1 and Gapdh protein levels of subcutaneous WAT. $*P< 0.05$, $**P< 0.01$.

Fig. 4 is a schematic illustration of DBZ-loaded PLGA microspheres enabling sustained release of DBZ to induce conversion of white adipocytes into beige adipocytes.

Fig. 5A is a fluorescent image of FITC-dextran-loaded microsphere. Scale bar 200 $\mu$m.

Fig. 5B is a SEM image of a population of DBZ-loaded PLGA microspheres. Scale bar 400 $\mu$m.

Fig. 5C is a higher magnification SEM image of a single DBZ-loaded PLGA microsphere. Scale bar 50 $\mu$m.

Fig. 5D is a size distribution of DBZ-loaded PLGA microspheres.

Figs. 6A and 6B are *in vitro* release profiles of FITC-dextran and DBZ from PLGA microspheres. Fig. 6A shows *in vitro* release of FITC-dextran from microspheres in PBS at pH 7.4 and 37°C. Fig. 6B shows *in vitro* release of DBZ from microspheres in PBS at pH 7.4 and 37°C.

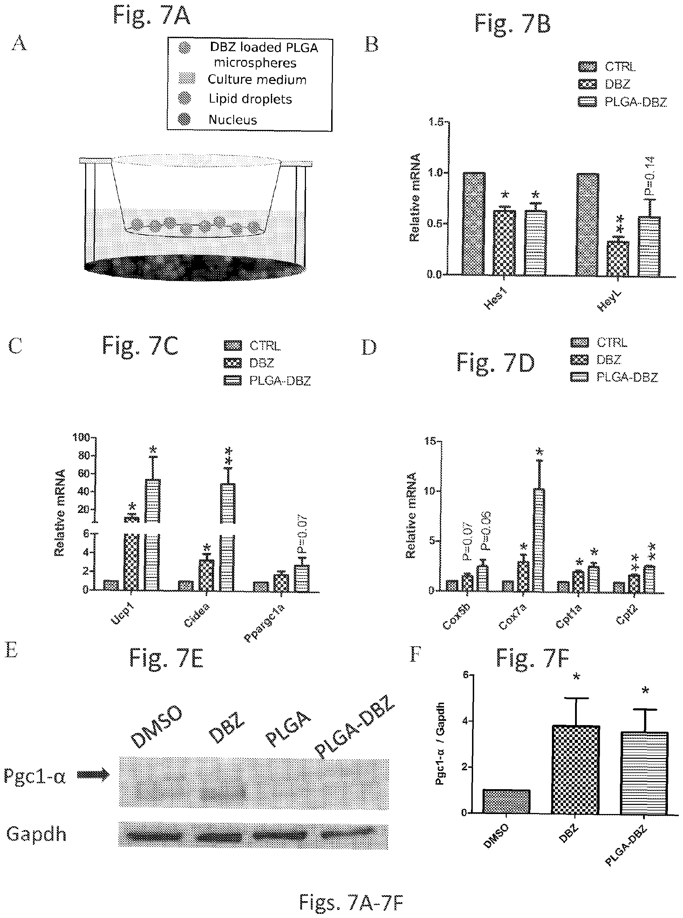

Figs. 7A -7F show the DBZ-loaded PLGA microspheres inhibit Notch and promote browning *in vitro*. Fig. 7A is a diagram of the co-culture system with primary white adipocytes seeded on well bottom and DBZ-loaded microspheres placed within a permeable insert. Mature adipocytes were labelled with BODIPY (red). Nuclei were counterstained with DAPI (green). Expression of (Fig. 7B) Notch target genes, (Fig. 7C) browning markers, and (Fig. 7D) mitochondria genes in white adipocytes co-cultured with DBZ-loaded PLGA microspheres. Fig. 7E shows protein levels of PGC1-$\alpha$ in white adipocytes co-cultured with DBZ-loaded PLGA microspheres. Fig. 7F shows quantification of protein level for Pgc1-$\alpha$ relative to Gapdh. DBZ: cells in DBZ-conditioned medium (10 $\mu$M); CTRL: cells treated with DMSO vehicle control. N=3. * P<0.05, ** P<0.01.

Fig. 8A is a fluorescence image showing the white adipocytes treated with DMSO (Green, MitoTracker; Red, Red C12. Scale bars, 200 $\mu$m).

Fig. 8B is a fluorescence image showing the white adipocytes treated with PLGA-DBZ microspheres (Green, MitoTracker; Red, Red C12. Scale bars, 200 $\mu$m).

Fig. 8C is a fluorescence image showing the white adipocytes treated with DBZ for 8 days (Green, MitoTracker; Red, Red C12. Scale bars, 200 $\mu$m).

Figs. 9A - 9D show characterization of FITC-dextran-loaded microspheres injection into mice. Fig. 9A shows direct injection of microspheres into inguinal adipose depot of WT mice. Fig. 9B shows injected microspheres (blue arrow) identified through gross observation of inguinal adipose tissues. Fig. 9C is a fluorescent image illustrating the distribution of microspheres after 24 hours of injection. Fig. 9D is a representative image showing green fluorescence from FITC-dextran-loaded microspheres dispersed in white adipocytes labelled by Oil Red O staining. Green: FITC; Red: Oil Red O.

Figs. 10A - 10C show DBZ-loaded PLGA microspheres induce browning *in vivo*. Fig. 10A shows H&E staining showing DBZ-loaded PLGA microspheres (yellow arrowhead) dispersed within the WAT depot. Scale bar, 200 $\mu$m. Fig. 10B shows H&E (top) and UCP1 (bottom) staining of inguinal WAT from mice at 14 days post injection of DBZ-loaded PLGA microspheres. Scale bars, 100 $\mu$m. Fig. 10C shows protein levels of Pgc1-$\alpha$ and Ucp1 of inguinal WAT from mice at 14 days post injection of DBZ-loaded PLGA microspheres.

Fig. 11 is a graph showing relative expression of adipocyte genes (values represent the mean of two biological replicates) after 14 days of DBZ-loaded microsphere injection.

Fig. 12 is a diagram showing an embodiment of the synthesis of PPHOS-$\gamma$-secretase inhibitor conjugate systems.

Fig. 13 illustrates adipocyte uptake of FTIC-BSA-loaded PS-*b*-(P4MVP)$_2$ polymersome nanoparticles. The nuclei were stained with DAPI. Green: FITC.

Figs. 14A- 14C show an embodiment of the polymersome structure. Fig. 14A is a TEM image of HPBD41-b-(P4MVP$_{25}$)$_2$ polymersome. Fig. 14B is a TEM image of 2% DBZ-loaded HPBD41-b-(P4MVP$_{25}$)$_2$ polymersome (scale bar: 50 nm). Fig. 14C is a TEM image of 5% DBZ-loaded HPBD41-b-(P4MVP$_{25}$)$_2$ polymersome (scale bar: 50 nm).

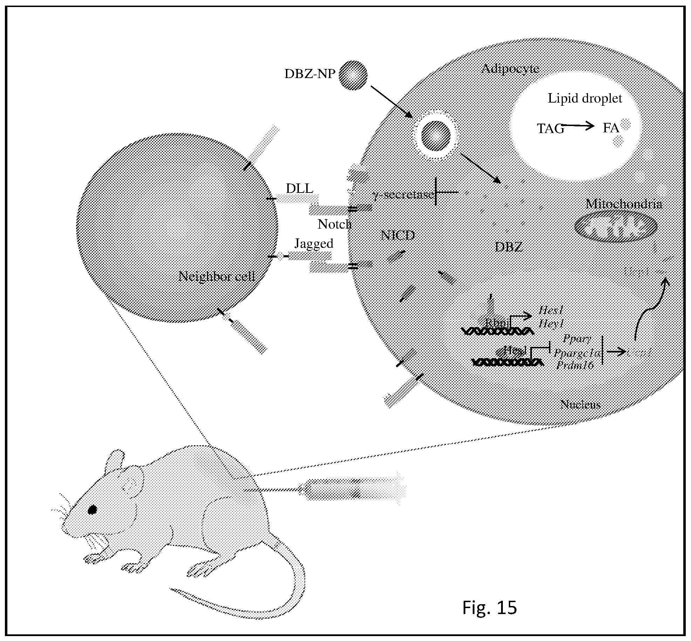

Fig. 15 shows a schematic illustration of DBZ-NPs intracellularly releasing DBZ to promote browning after local injection into inguinal WAT.

Fig. 16 illustrates **Nanoparticles fabrication and characterization. (a)** Schematic illustration of synthesis of DBZ-NPs by nanoprecipitation method. The morphology of DBZ-NPs was characterized by TEM. Scale bar, 200nm. **(b)** *In vitro* release profiles of DBZ from DBZ-NPs at pH 5.0 and 7.4 conditions.

Fig. 17 illustrates *In vitro* **cellular interaction with nanoparticles. (a)** Cellular uptake of nile red-loaded PLGA nanoparticles in primary pre-adipocytes within 10 (top) and 60 (bottom) min. The late endo/lysosomes were stained with LysoTracker Green (LTG) and the nuclei were stained with Hoechst 33342. Scale bar, 20$\mu$m. **(b)** Representative TEM images of nanoparticles in the endocytic vesicle (left) and endosome (right). The arrows point to the nanoparticles. Scale bar, 500nm.

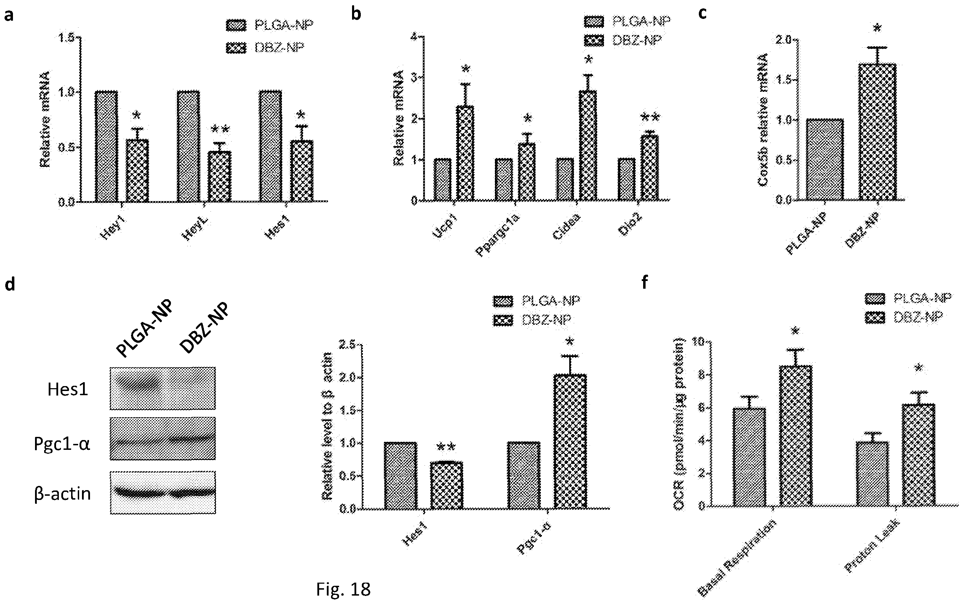

Fig. 18 illustrates **DBZ-NPs induce browning *in vitro*.** SVF cells were isolated from inguinal WAT and were treated with DBZ-NPs during adipogenic differentiation. **(a, b)** Quantitative PCR analysis showing the mRNA levels of Notch target genes **(a)** and browning marker genes **(b)** in differentiated adipocytes treated with PLGA-NPs and DBZ-NPs. **(c)** Representative western blot results showing the protein expression levels of Hes1 and Pgc-1$\alpha$ in differentiated adipocytes treated with PLGA-NPs and DBZ-NPs. **(d)** Quantification of protein levels of Hes1 and Pgc-1$\alpha$ normalized to $\beta$-actin controls. **(e)** Quantitative PCR analysis showing the mRNA levels of *Cox5b* in differentiated adipocytes treated with PLGA-NPs and DBZ-NPs. **(f)** OCR under the conditions of basal respiration and proton leak normalized to total protein from differentiated adipocytes. Data are shown as the means ± s.e.m. n=3 mice for each group. * P< 0.05, ** P<0.01.

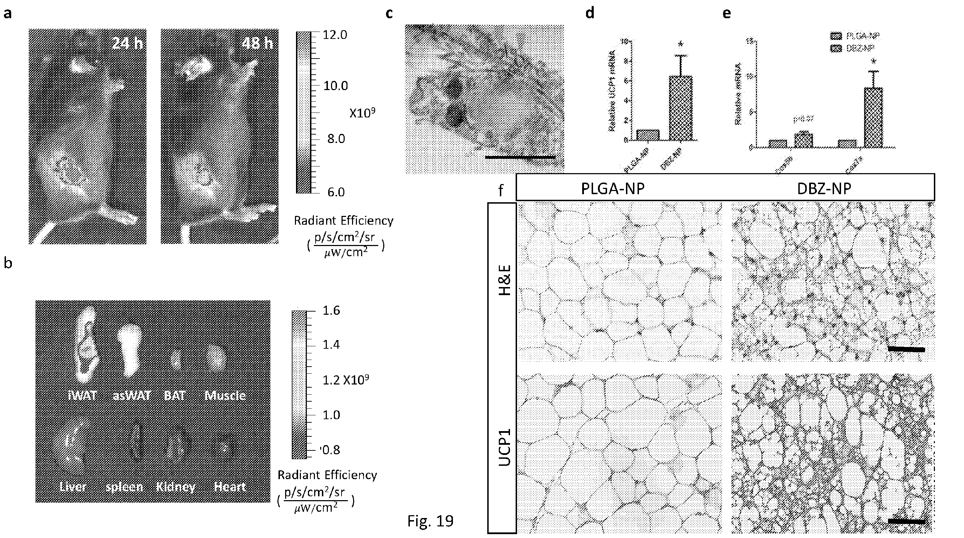

Fig. 19 illustrates **the local retention of DBZ-NPs promote the browning of inguinal WAT. (a)** *In vivo* fluorescence imaging at 24 and 48 h after local injection of PLGA-cy5.5 nanoparticles into inguinal WAT depot. **(b)** *Ex vivo* fluorescence imaging of different tissues at 72 h post injection. **(c)** Representative TEM image showing that the nanoparticles were localized inside the adipocyte. The arrows point to the nanoparticles. The dot line and the arrowheads indicate the cell membrane and membrane vesicles, respectively. Scale bar, 500 nm. **(d, e)** Quantitative PCR analysis showing the mRNA levels of *UCP1* **(d)** and mitochondria genes, *Cox5b and Cox7a* **(e),** from mice treated with PLGA-NPs and DBZ-NPs. Data are shown as the means ± s.e.m. n=7 mice for each group. * P< 0.05. **(f)** Representative images of H&E and UCP1 staining of inguinal WAT after treatment with PLGA-NPs and DBZ-NPs. Scale bar, 50 μm.

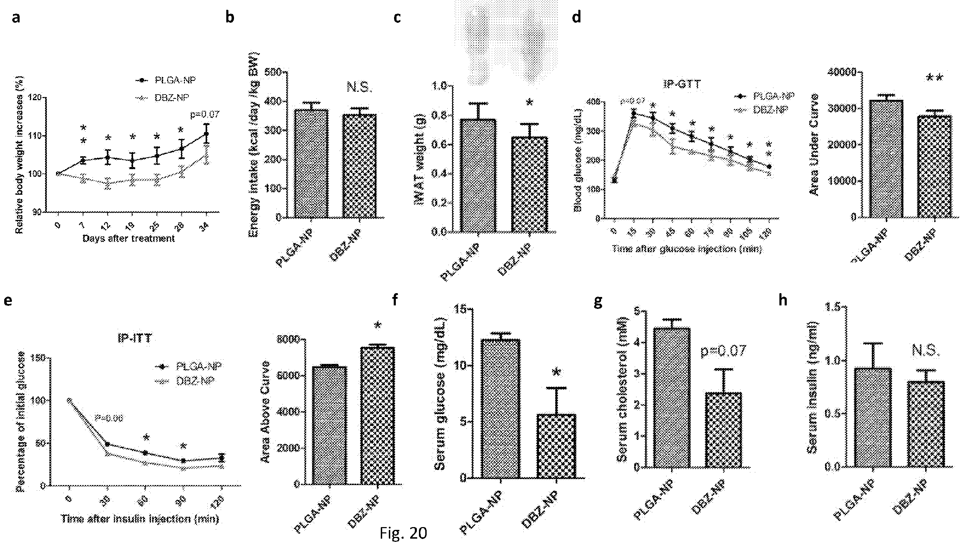

Fig. 20 illustrates **DBZ-NPs prevent diet-induced obesity. (a)** Relative body weight increases of mice receiving PLGA-NPs and DBZ-NPs after normalization to initial body weight, **(b)** Average energy intake per mouse per day after normalization to body weight, **(c)** The weight of inguinal WAT with treatment of PLGA-NPs and DBZ-NPs was quantified. Representative images are shown. **(d)** Blood glucose concentration and area under curve quantification during glucose tolerance test performed on the mice receiving PLGA-NPs and DBZ-NPs. **(e)** Blood glucose concentration and area above curve quantification during insulin tolerance test performed on the mice treated with PLGA-NPs and DBZ-NPs. **(f, g, h)** Quantification of serum levels of glucose **(f)**, cholesterol **(g),** and insulin **(h)** from fasting mice treated with PLGA-NPs and DBZ-NPs. Data are presented as the means ± s.e.m. n≥4 mice for each group. * P< 0.05, ** P<0.01, N.S. non-significant.

Fig. 21 illustrates **Mathematical modeling of the DBZ release profiles.** The *in vitro* release profiles of DBZ from DBZ-NPs fit to the Korsmeyer-Peppas model at pH 5.0 (a) and 7.4 (b). Data are shown as the means ± s.e.m. n=3 for each group.

Fig. 22 illustrates **Cellular uptake of nanoparticles** *ex vivo.* **(a)** Representative image showing the cellular localization of nile red-loaded nanoparticles in mature adipocytes. Nuclei were stained with Hoechst. **(b)** Representative TEM image showing a number of nanoparticles inside the adipocyte. The arrows point to the nanoparticles. The dot line indicates the cell membrane. LD, lipid droplet. Scale bar, 2μm.

Fig. 23 illustrates **Serological parameters from fasting mice treated with PLGA-NPs and DBZ-NPs. (a,b)** Quantification of serum levels of triglyceride **(a)** and free fatty acids **(b)** from fasting mice treated with PLGA-NPs and DBZ-NPs. **(c)** Insulin resistance was calculated as insulin X free fatty acids. Data are presented as the means ± s.e.m. n≥4 mice for each group. * P< 0.05, N.S. non-significant.

Fig. 24 illustrates **Fatty acid metabolism in the liver of mice receiving PLGA-NPs and DBZ-NPs. (a)** Representative images of Oil Red O staining of liver sections from mice receiving PLGA- and DBZ-NPs. Scale bar, 20μm. **(b,c,d)** qPCR analysis showing the mRNA levels of *Cpt1* **(b)**, Atgl and Hsl **(c),** and *Acc1* **(d)** in the livers of mice treated with PLGA-NPs and DBZ-NPs. Data are presented as the means ± s.e.m. n≥4 mice for each group. * P< 0.05.

Table 1 **Optimized parameters for 100mg nanoparticle synthesis.**

Table 2. **Mathematical model examination for the DBZ release from nanoparticles at pH 5.0 and 7.4.** $R^2$-adjusted, Akaike Information Criterion (AIC) and the Model Selection Criterion (MSC) employed for model selection (n=3). The best model possesses the highest value of $R^2$-adjusted, the lowest AIC value and the largest MSC.

Table 3. **qPCR primers used in this study.**

## BRIEF DESCRIPTION OF SEQUENCE LISTING

SEQ ID NO:1 and 2 are forward and reverse qPCR primers for Hey1.
SEQ ID NO:3 and 4 are forward and reverse qPCR primers for HeyL.
SEQ ID NO:5 and 6 are forward and reverse qPCR primers for Hes1.
SEQ ID NO:7 and 8 forward and reverse qPCR primers for UCP1.
SEQ ID NO:9 and 10 are forward and reverse qPCR primers for Ppargc1a.
SEQ ID NO:11 and 12 are forward and reverse qPCR primers for Cidea.
SEQ ID NO:13 and 14 are forward and reverse qPCR primers for Dio2.
SEQ ID NO:15 and 16 are forward and reverse qPCR primers for Cox5b.
SEQ ID NO:17 and 18 are forward and reverse qPCR primers for Cox7a.

## DETAILED DESCRIPTION

For the purposes of promoting and understanding of the principles of the present disclosure, reference will now be made to the embodiments illustrated in the drawings, and specific language will be used to describe the same. It will nevertheless be understood that no limitation of the scope of this disclosure is thereby intended.

In response to the unmet need, disclosed herein are novel therapeutic methods and systems based on a design of polymeric drug delivery systems to induce browning of adipocytes for treatment and/or prevention of obesity and its affiliated metabolic symptoms.

The global epidemic of obesity and its associated risks of chronic diseases affect > 10% of the world's population. Currently, there are few medications available for obesity treatment. Mostly, those therapies are dedicated to decreasing energy intake by either suppressing appetite through stimulating the central nervous system or reducing nutrient digestion and absorption within the gastrointestinal tract. However, those medications only produce modest effects and are usually accompanied with unpleasant, potentially harmful side effects. Thus, development of alternative effective treatment strategies is warranted.

Obesity is mainly due to systemic energy surplus that is stored as lipids in white adipocytes, whose expansion leads to obesity. Brown adipocytes prominently found in most mammalian species primarily function to dissipate energy surplus (lipids) to generate heat, thus increasing energy expenditure and counteracting obesity. The relatively identified beige adipocytes present in white adipose tissues (WAT) are inducible 'brown-like' cells, which can be bi-directionally converted from and to white adipocytes under the control of environmental temperature or sympathetic nerve innervation. Elevated expression of uncoupling protein 1 (Ucp1), a key regulator of thermogenesis in cold-acclimated beige adipocytes, is associated with robust uncoupled respiration and thermogenesis. Notably, the activities of beige adipocytes correlate with leanness in humans. Thus, transformation of white adipocytes into beige adipocytes (browning) holds great promise to develop new therapeutics to raise energy expenditure and reduce adiposity.

Current strategies for enhancement of beige adipocyte biogenesis and function involve genetic and pharmacological manipulations. However, these methods are often associated with the risks of having unintended adverse consequences on other cells/tissues, and offer little control over the location and temporal extent of beige adipocyte biogenesis. For example, administration of fibroblast growth factor 21 (FGF-21) is associated with systemic side effects on bone loss.

Polymer-based drug delivery systems enable sustained, spatio-temporally controlled drug release, and offer several unique advantages over conventional drug delivery including continuous drug release, decreased systemic side effects, and increased patient compliance. The drug release profiles can be fine-tuned by modulating characteristics of polymers such as material chemistry, molecular weight, hydrophobicity/hydrophilicity, surface charge, degradation and erosion mechanisms. Several examples of approved products by the Food and Drug Administration (FDA) include GLIADEL Wafer, drug-eluting stents, drug-releasing transdermal patches, and absorbable sponges or meshes impregnated with antibiotics. In this disclosure we provide a polymer-based drug delivery platform to induce browning of local WAT for treatment of obesity and its related metabolic syndromes. The polymeric delivery platform enables controlled and sustained release of effective drugs, particularly of the Notch inhibitors, for inducing browning of adipocytes following administration of compositions containing a polymer to the WAT depots. The effective drug for this effect may be a yet to be discovered compound or composition that induces browning of adipocyte locally aided with the utilization of the polymer. A non-limiting category is any compound or composition that is capable of inducing browning of WAT. Exemplary compounds and compositions are provided in details below.

The drug can be a therapeutic agent that induces formation of brown and/or beige adipocytes, such as β-adrenergic activators (e.g. CL316, 243 and mirabegron), catecholamines (e.g. norepinephrine), PPAR-y agonists (e.g. rosiglitazone and thiazolidinediones), Capsinoids and capsinoid-like compounds (e.g. grains of paradise extracts), adipose inflammation related cytokines (e.g. IL4), natriuretic peptides (e.g. atrial natriuretic peptide (ANP), brain-type natriuretic peptide (BNP) and C-type natriuretic peptide (CNP)), FGF family members (e.g. FGF1, 15/19,21), BMP family members (e.g. BMP7, BMP8b), thyroid hormones (e.g. T3 and T4 thyroid hormones) and their receptor agonists (e.g. T3 receptor agonists), myokines (e.g. Irisin and meteorinlike (METRNL)), VEGF family members (e.g. VEGF-A), Notch inhibitors (e.g. γ-secretase inhibitors), Janus kinase inhibitors (e.g. tofacitinib), spleen tyrosine kinase inhibitors (e.g. R406), prostaglandins and their synthetic analogs, cyclooxygenase-2 agonists, retinoic acid and retinaldehyde, retinaldehyde dehydrogenases activators, adenosine and its receptor activators, parathyroid hormone-related protein (PTHrP), adipokine (e.g. neuregulin 4, adiponectin), orexin, and microRNAs/siRNAs targeting the aforementioned factors related signaling pathways.

A new role of Notch signaling in regulating the homeostasis of white and beige adipocytes is disclosed. Inhibition of Notch signaling through intraperitoneal injection of dibenzazepine (DBZ), a γ-secretase inhibitor has been found to induce browning of white adipocytes and consequent reduced obesity and improved glucose balance in obese mice. Notch inhibitors useful in the present invention as effective drugs are γ-secretase inhibitors, for example dibenzazepine (DBZ) and (N-[N-(3,5-difluorophenacetyl)-1-alanyl]-S-phenylglycine t-butyl ester) (DAPT).

The drug should be incorporated into the polymer in the compositions of the invention in a percent loading of 0.1% to 70% by weight. The drug release kinetics can be tailored by manipulating the percent drug loading, the nature of polymer, and the method used for making and loading the delivery system.

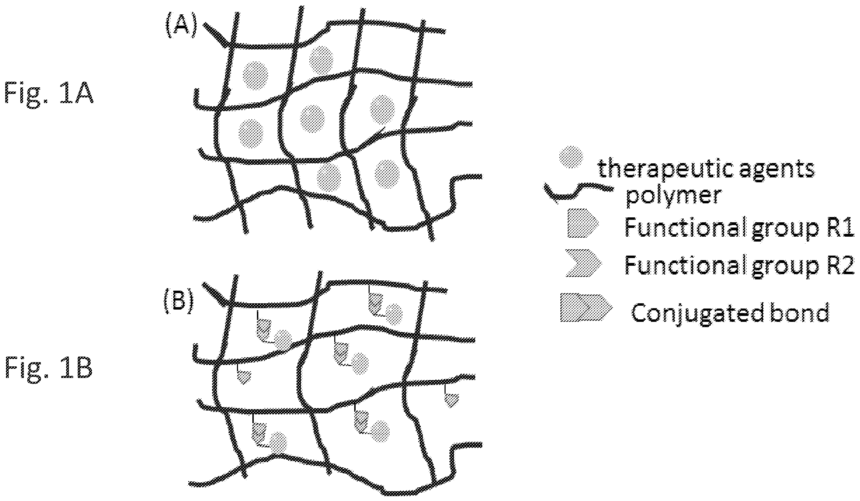

The disclosure includes methods for making a polymer-based drug delivery system via combining a drug with a polymer matrix system. The polymer-based drug delivery system can be formulated from encapsulation of therapeutic drugs within a polymer matrix system through physical interactions (Fig. 1A). The physical interactions include, but are not limited to hydrophobic interaction, hydrophilic interaction, hydrogen bonding, and inter-molecular electrostatic interactions. The polymer-based drug delivery system can be also formulated from encapsulation of therapeutic drugs through chemical interactions (e.g. polymer-drug conjugation) within a polymer matrix system (Fig. 1B). Polymer-therapeutic conjugation reactions include, but are not limited to, amine reaction, thiol reactions (e.g. thiol click reactions), carboxylate reaction, hydroxyl reactions, aldehyde and ketone reactions, active hydrogen reactions, photo-chemical reaction, and cycloaddition reactions (e.g. Diels-alder reaction, copper-catalyzed azide-alkyne cycloaddition (CuAAC), copper-free azide-alkyne huisgen cycloaddition). The functional groups R1 and R2 can include but are not limited to, amines, thiol, carboxylic acid, aldehyde, ketone, active hydrogen sites on aromatic ring, diene, azide isothiocyanates, isocyanates, acyl azides. N -hydroxysuccinimide (NHS) ester, sulfo-NHS, sulfonyl chloride, aldehydes, epoxides, carbonates, aryl halide, imidoesters, carbodiimides (e.g. N, N'-dicyclohexylcarbodiimide (DCC) and 1-Ethyl-3-(3-dimethylaminopropyl) carbodiimide (EDC)), alkylphosphate compound, anhydride, fluorophenyl ester, hydroxymethyl phosphine, guanidino group, iodoacetyl derivative, maleimides, aziridines, acryloyl derivatives, arylating agents, disulfide derivative, vinylsulfone, phenylthioester, cisplatin, diazoacetate, carbonyl diimidazole, oxiranes, N, N'-disuccinimidyl carbonate (DSC), N-hydroxylsuccinimidyl chloroformate, alkyl halogens, hydrazine, maleimide, alkyne, phosphorus-bound chlorine. The conjugated linkages can include but are not limited to, isothiourea, isourea, amide, sulfonamide, shift-base, secondary amine, carbamate, arylamine, amidine, phosphoramidate, thioether, disulfide, $\beta$-thiosulfonyl, ester, carbamate, hydrazone, diazo, 2+4 cycloaddition, 1,2,3-triazoles, carbohydrates, amino acid esters bond.

The polymer matrix system can comprise, consist of, or consist essentially of synthetic polymers. Synthetic polymers include, but are not limited to, poly (aliphatic ester) (e.g. poly(lactide) (PLA), poly($\varepsilon$-caprolactone) (PCL), poly(glycolic acid) (PGA), poly(lactic-co-glycolic acid) (PLGA), poly(trimethylene carbonate) (PTMC), polydioxanone (PDS), poly(ortho ester), polyanhydrides, poly(anhydride-co-imide), poly(anhydride-esters), polyurethanes (e.g. Degrapols), poly(glycerol sebacate), poly(ethylene imine), poly(acrylic acid) (PAA), polyethylene glycol (PEG), poly(vinyl alcohol) (PVA), poly(N-isopropylacrylamide) (PNIPAm), poly(oxazolines) (e.g. poly(2-methyloxazoline and poly(2-ethyl-2-oxazoline), oligo(ethylene glycol) fumarates (OPFs), poly(propylene fumarate), poly(alkyl cyanoacrylates), polyacrylic amide, synthetic poly(amino acids) (e.g. poly (L-glutamic acid) (L-PGA) and poly (aspartic acid)), polyphosphazenes, poly(phosphoesters), lipidoids, and blends thereof. In some embodiments, the synthetic polymers can be PLGA with a 50:50 ratio of lactic to glycolic acid.

Further, the polymer matrix system can include natural polymers. The natural polymers can include, but are not limited to, fibrin, collagen, matrigel, elastin, elastin-like peptides, albumin, natural poly (amino acids) (e.g. cyanophycin, poly ($\varepsilon$-L-lysine) and poly (x-glutamic acid)), and polysaccharides (e.g. hyaluronic acid, chitosan, dextran, chondroitin sulfate, agarose, alginate, and heparin), lipids, and blends thereof.

The polymer matrix system can include non-degradable polymers. Non-biodegradable polymers include, but are not limited to, poly(ethyl ethylene) (PEE) , poly(butadiene) (PBD), poly(dimethylsiloxane) (PDMS), and poly(styrene) (PS), poly (N-ethyl-4-vinypyridinium), poly(2,2-(dimethyl aminoethyl methacrylate), poly(ethylene imine), poly(allylamine), and poly(diallyl dimethyl ammonium chloride), poly(acrylic acid), poly(styrene sulfonate), poly(vinyl sulfate), and poly(3-sulfopropyl methacrylate), and polyacrylic amide and blends thereof.

The polymer matrix system can include polymer composites. For example, polymer-polymer blends may be formulated to synergistically combine the beneficial properties of parent polymers. Composites combining a mixture of polymers having different release rates can be obtained to achieve a desirable release profile.

The polymer matrix system can include polymer conjugates. Direct conjugation of drug onto polymer backbone helps to ensure a high drug loading efficiency and sustained drug release at the target site. Drug release kinetics can be tailored by changing the nature and hydrolytic sensitivity of the polymer-drug linkage. Such a polymer-drug conjugate system can also be used to develop three-dimensional matrices to either study cell-extracellular microenvironment interactions or generate engineered brown fat for transplantation.

The polymer matrix system can include, but are not limited to linear, star-shaped, hyper-branched, and crosslinked architectures.

The polymer matrix system can further comprise one or more surface modifications including, but are not limited to, PEGylation or the use of ligands targeting receptors on adipocytes.

The disclosure also provides a method of administering the claimed polymer delivery system compositions to a subject by injection or surgical implantation at the site where browning of fat is to be achieved. Local delivery allows a therapeutic concentration of the drug to be delivered to the WAT depots with minimized systemic side effects. The method can also include the step of administering a therapeutic amount of the drug to induce browning of adipocytes within the subject. In yet another embodiment, a method of treating obesity in a subject is presented. The polymer system can be of any size suitable for delivery to the site in the form of slabs, beads, pellets, hydrogels, microparticles, nanoparticles, or their combination. The polymer drug systems can be fabricated by a number of well-established techniques including solvent casting, oil-in-water emulsion/solvent evaporation, spray drying, or precipitation using a solvent/nonsolvent system. In the preferred embodiments, the polymer system will be in the form of particles. For example, in some aspects, the polymer system comprises microparticles that are configured for injection into the WAT of a subject. In other aspects, the polymer system comprises nanoparticles that are configured for injection into the WAT of a subject.

Adipocytes, adipocyte progenitor cells, and stem cells can be combined with the polymer matrix system to engineer brown/beige adipose tissues as a novel therapeutic to treat obesity and related disorders using scaffold-based tissue engineering principles. In one embodiment, cells can be harvested from the subjects through fat tissue biopsy. The cells can then be seeded onto the three-dimensional polymeric scaffolds mimicking the natural extracellular matrices. Following *in vitro* culture, the cell/scaffold constructs can be transplanted back to the subjects. A variety of chemical and biological factors (e.g. browning agents), and mechanical stimuli (e.g. bioreactor) can be applied to facilitate the tissue generation process prior to transplantation. In one embodiment, a biodegradable Notch inhibitor-loaded PLGA microsphere system is disclosed. Biodegradable polymer microspheres with predetermined degradation and drug release profiles are a focus for development of effective drug delivery systems for a variety of applications including cancer, cardiovascular disease, and vaccine development. Among a variety of polymers that have been employed for drug delivery, PLGA, copolymers of lactic acid and glycolic acid, have been particularly prominent due to biocompatibility, biodegradability, and convenient processability. Drug release kinetics of PLGA microspheres can be controlled by modulating the microsphere size, molecular weight and composition of PLGA.

In one embodiment, a biodegradable polyphosphazene-$\gamma$-secretase inhibitor conjugate system is disclosed. Polyphosphazenes (PPHOS) having a backbone of alternating phosphorus and nitrogen atoms represent a unique material class for drug delivery due to synthetic flexibility, tunability of polymer properties, and biocompatibility. Each phosphorus atom bears two organic substituents, with a variety of side groups available for property optimization. The release of side groups resulting from the PPHOS degradation enables the rational design of PPHOS-based delivery systems using the pro-drug concept. Drug molecules such as $\gamma$-secretase inhibitors can be directly attached as side groups onto the polymer chain using hydrolytically sensitive bonds as depots for drug delivery.

In another embodiment, a nanoparticle-based drug delivery system comprising polymersomes is disclosed. Polymersomes are a class of artificial vesicles made from synthetic amphiphilic block copolymers. Typical polymersomes are hollow spheres that contain an aqueous solution in the core surrounded by a bi-layer membrane. The bi-layer membrane is composed of hydrated hydrophilic coronas both at the inside and outside of the hydrophobic middle part of the membrane separating and protecting the fluidic core from the outside medium. Block copolymers comprise two or more homopolymer blocks. Each block is polymerized with a specific monomer or a combination of monomers that have unique physico-chemical properties in the polymers. In those embodiments the hydrophobic block include, but are not limited to, non-biodegradable poly(ethyl ethylene) (PEE), poly(butadiene) (PBD), poly(dimethylsiloxane) (PDMS), and poly(styrene) (PS) as well as biodegradable poly(lactide) (PLA), poly($\varepsilon$-caprolactone) (PCL) and poly(trimethylene carbonate) (PTMC) etc.; In those embodiments the hydrophophilic block include, but are not limited to, poly (N-ethyl-4-vinypyridinium), poly(2,2-(dimethyl aminoethyl methacrylate), poly(ethylene imine), poly(allylamine), poly(diallyl dimethyl ammonium chloride), poly(acrylic acid), poly(styrene sulfonate), poly(vinyl sulfate), poly(3-sulfopropyl methacrylate), polyethylene glycol, poly(2-methyloxazoline), poly(2-ethyl-2-oxazoline), and polyacrylic amide etc. The polymersomes can be utilized for encapsulation of therapeutic molecules through physical interaction (e.g. hydrophobic interaction, hydrophilic interaction, hydrogen bonding, and inter-molecular electrostatic interactions) or through chemical interactions (e.g. polymer-drug conjugation) within a polymer. The aqueous core of polymersomes can be utilized for the encapsulation of hydrophilic therapeutic molecules. The membrane can integrate hydrophobic drugs within its hydrophobic core.

In another embodiment, a biodegradable nanoparticle-based drug delivery system (DBZ-NPs) comprising PLGA and DBZ is disclosed by leveraging the discovery of the role played by Notch signaling in adipocyte plasticity and benefits of nanoparticle-based intracellular drug release in enhancing the therapeutic efficacy and minimizing potential side effects. It is possible to enable cell- or tissue-specific delivery with systemic administration by conjugating targeting moiety to nanoparticles. For example, the interaction between one specific peptide CKGGRAKDC (SEQ ID NO:20) and prohibitin, a vascular marker in the adipose tissue, has been reported to facilitate nanoparticle targeting to the vasculature in the adipose tissues.

In alternate embodiments, other polymer systems that can be utilized for delivery of drugs (for example, browning agents) include but are not limited to nanocarriers, microparticles, hydrogels, fiber matrices, wafers or films, capsules, patches, and implantable drug delivery devices.

Although the application to obesity is mentioned in this disclosure, it should be appreciated that such application is not intended to be limiting on the applications of the herein described method and systems. Such an enabling polymer-based technology platform can be readily adapted to treat a broad spectrum of diseases such as fatty liver disease and atherosclerosis.

Importantly, we have demonstrated that the local delivery of DBZ-NPs prevented HFD-induced obesity and improved glucose homeostasis as well as fatty acid metabolism in the liver, verifying the direct regulatory effects of Notch inhibition in WAT on the systemic metabolic profiles. These systemic benefits elicited by the local manipulation are in agreement with the previous publication that the transplantation of either subcutaneous WAT from exercise-trained mice or BAT from normal mice significantly improved glucose tolerance and insulin sensitivity[25, 26]. It remains elusive whether these metabolic benefits are purely due to the elevated uptake of glucose and fatty acids or additionally involve other endocrine factors secreted by transformed beige adipocytes.

**EXAMPLE 1: *Inhibition of Notch signaling promote browning of white adipocytes:***

The Notch signaling pathway is important for cell-cell communication and cell-fate determination during development and is required for adult tissue homeostasis. Notch target gene Hairy/enhancer-of-split 1 (Hes1) directly binds to the promoters of PR domain-containing 16 (*Prdm16*), peroxisome proliferator-activated receptor gamma (*Pparγ*), and *Pparγ* coactivator 1 alpha (*Ppargc1a*) to inhibit their transcription. This leads to reduced mitochondrial biogenesis and reduced levels of Ucp1.

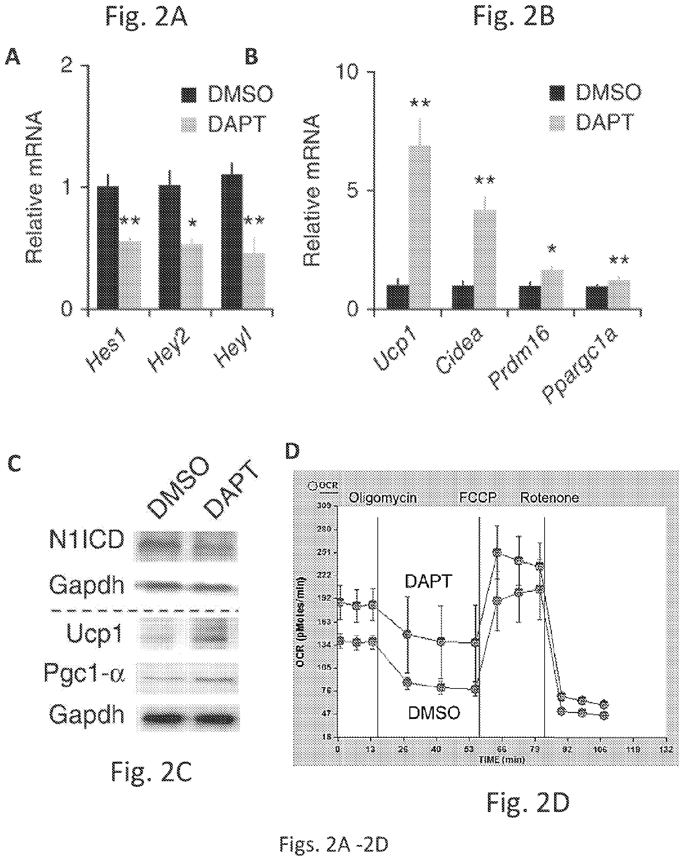

Pharmacological inhibition of Notch signaling induces browning of white adipocytes during *in vitro* culture. Notch signaling is inhibited in cultured white adipocytes with the γ-secretase inhibitor DAPT (N-[N-(3,5-difluorophenacetyl)-1-alanyl]-S-phenylglycine t-butyl ester). DAPT inhibited Notch signaling and upregulated the expression of brown fat specific genes Ucp1, Cidea, Prdm16 and Ppargc1a (Figs. 2A-2C). Moreover, adipocytes treated with DAPT exhibited a higher oxygen consumption rate (OCR) than those treated with DMSO, as is expected if browning occurs (Fig. 2D).

WAT is the primary site of long-term energy storage in most vertebrate species. In response to excess calorie intake, the size of the WAT expands through hyperplasia and hypertrophy of adipocytes. In response to exposure to cold ambient temperatures, the sympathetic nervous system releases catecholamines, which bind β-adrenoceptors and activate lipolysis through the cAMP pathway. Fatty acids generated from lipolysis can directly activate Ucp1 for heat production. Understanding the pathways that effectively function to regulate adipocyte homeostasis is, thus, fundamental to the treatment of obesity. It has been recently reported that Notch signaling plays a role in regulating the plasticity (conversion) of white and beige adipocytes *in vivo,* consequently affecting body energy metabolism. Adipocyte-specific ablation of *Notch1* or *Rbpj* driven by aP2-Cre results in decreases in the size of various adipose depots and increases in the relative abundance of beige adipocytes in WAT, accompanied by increased metabolic rate, improved glucose tolerance, and insulin sensitivity in experimental animals. These phenotypes are associated with elevated levels of expression of beige adipocyte-specific genes in WAT but not BAT. In addition, mice depleted of *Notch1* or *Rbpj* exhibit accelerated browning (appearance of beige adipocytes within WAT) in response to cold environments. The adipose-specific Notch1 mutant mice are also resistant to HFD-induced obesity. In initial efforts to confirm the role of Notch signaling in adipose tissues, activation of Notch signaling using a highly adipocyte-specific adiponectin-Cre mouse was shown to inhibit browning of WAT and induce whitening of BAT, manifested by lipid deposition and the emergence of white adipocytes in the classic interscapular BAT. Adiponectin-Cre-induced Notch activation also rendered mice to become glucose intolerant and insulin resistant. These phenotypes are in sharp contrast to those observed in Notch-deficient mice.

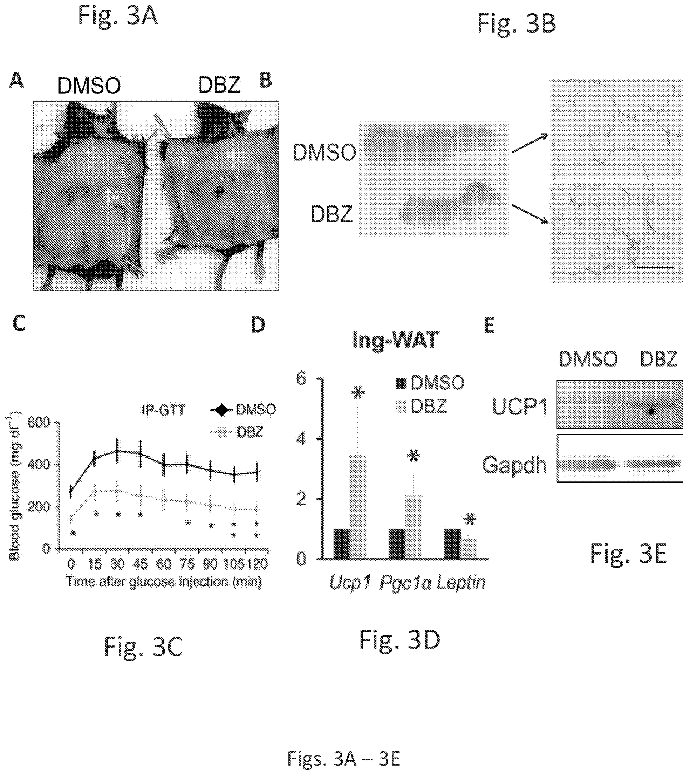

To assess the extent to which Notch inhibition ameliorates obesity *in vivo* under pathological conditions, ob/ob mice deficient in Leptin (*Lep*ob) were treated with dibenzazepine (DBZ, a γ-secretase inhibitor). Notably, DBZ treatment attenuated the body-weight gain seen in the vehicle-treated control group without affecting energy intake. The body-weight differences were associated with smaller size and less weight of various WAT depots in the DBZ-treated compared to vehicle-treated mice (Fig. 3A,B). DBZ-treated mice also exhibited improved glucose tolerance and insulin sensitivity relative to controls (Fig. 3C). Consistent with reduced adiposity, DBZ-treated mice showed a lower respiration exchange ratio compared with control mice, indicating that DBZ administration initiated a metabolic shift toward the utilization of fat as a relevant energy source. At the molecular level, DBZ administration resulted in the inhibition of the expression of the Notch targets *Hes1, Hey1* and *HeyL,* as well as *Lep,* but upregulated the expression of *Ucp1* and *Ppargc1a* in WAT (Fig. 3D, E). DBZ administration also resulted in suppression of expression of the inflammatory cytokines encoded by *Cd68, Il6* and *Il1b* that are associated with obesity in humans. At the completion of the experiment, fed blood glucose concentrations remained low in the presence of DBZ injection, suggesting a long-term beneficial effect of adipose browning on glucose metabolism.

However, potential compliance issues associated with requirements for multiple periodic drug injections as well as safety and efficacy concerns over widespread drug distribution in the body raise significant hurdles to the clinical translation of this discovery. Thus, there is a critical need for the development of new drug delivery systems that would allow selective spatio-temporal delivery of Notch inhibitors such as DBZ to adipose tissues.

**EXAMPLE 2: *PLGA microsphere-based DBZ delivery system to promote browning of WAT for the treatment of obesity:***

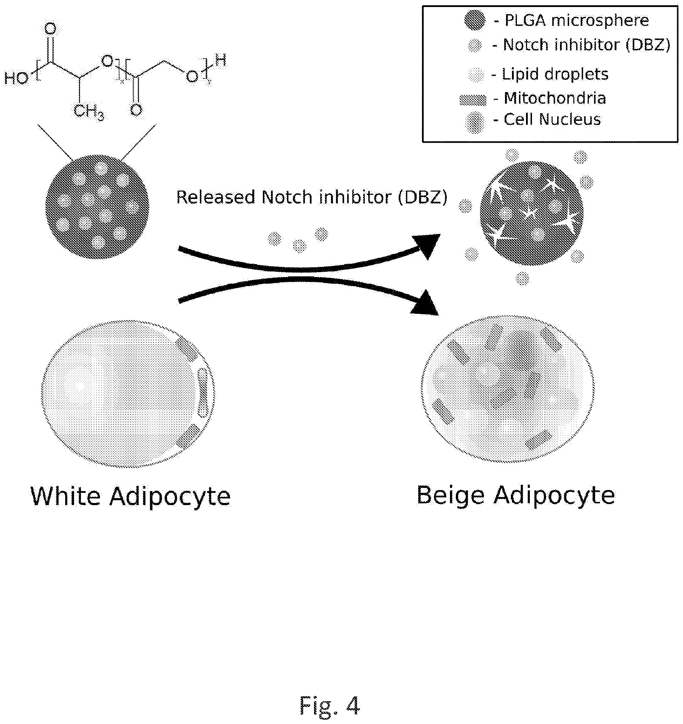

In this example, a PLGA microsphere-based DBZ delivery system to promote browning of WAT for the treatment of obesity is disclosed (Fig. 4). PLGA with a 50:50 lactide to glycolide ratio was utilized since this specific polymer has a relatively fast degradation rate among the PLGA family, which is advantageous to induce browning. We formulated the DBZ-loaded PLGA microspheres using an emulsion/solvent evaporation technique and characterized the morphology and release profiles of those microspheres. Moreover, we demonstrated that the DBZ-loaded PLGA microspheres inhibited Notch and consequently promoted browning both *in vitro* and *in vivo.* To our knowledge, this is the first time that an effective bioengineered system was developed to deliver therapeutic agents to convert white adipocytes to beige adipocytes. This study not only contributes to our understanding of the underlying mechanism of Notch inhibition in the browning process but also paves the way for development of novel therapeutic strategies to counteract obesity and its associated metabolic syndrome in humans.

**Materials and Methods:**

*Materials:*

PLGA (lactide:glycolide 50:50; Mw=75,000) was obtained from Lakeshore Biomaterials. Poly(vinyl alcohol) (PVA; 87-90% hydrolyzed, average Mw=30,000-70,000), fluorescein isothiocyanate labeled dextran (FITC-dextran) (Mw=70,000), and DBZ were obtained from Sigma. Methylene chloride (DCM), dimethyl sulfoxide (DMSO), and acetonitrile were purchased from Fisher Scientific.

*Animals:* All the mice used in this study were wild-type lean mice kept under normal maintenance in a clean facility. All procedures regarding animal maintenance and experimental use were conducted following regulations.

*FITC-dextran-loaded microsphere formulation and characterization*

FITC-dextran-loaded PLGA microsphere were prepared by a water-in-oil-in-water (WOW) double emulsion technique. Briefly, 0.4 g of PLGA was dissolved in 4 mL DCM. Into this organic phase (O), 330 $\mu$l of aqueous solution (W1) containing ~3.7 mg of FITC-dextran was emulsified using a vortex mixer operating at 1,000 rpm for 3 min to form the W1/O emulsion. This primary emulsion was injected into 400 mL of an aqueous phase containing 1% (w/v) PVA (W2). The resulting W1/O/W2 emulsion was stirred at 400 rpm for overnight with an overhead magnetic stirrer to allow solvent evaporation and microspheres hardening. The microspheres were then isolated by centrifugation, washed three times with distilled water, and were dried in a vacuum oven for 24 hours. Final products were stored in a desiccator. FITC-dextran-loaded PLGA microspheres were imaged by fluorescence microscopy (EVOS FL).

*DBZ-loaded microsphere formulation and characterization*

PLGA microspheres loaded with DBZ were prepared by an oil-in-water (O/W) emulsion/solvent evaporation technique. The oil phase had 540 mg of PLGA and 10.8 mg of DBZ dissolved in 2.7 mL DCM. This oil phase was injected into 400 ml of an aqueous phase containing 1% (w/v) PVA, which was stirred at 600 rpm to achieve an O/W emulsion system. The resulting emulsion was stirred overnight with an overhead magnetic stirrer to allow complete evaporation of the solvent and solidification of the droplets into microspheres. The microspheres were then isolated by centrifugation, washed three times with distilled water, and dried in a vacuum oven for 24 hours. Final products were stored in a desiccator. The microsphere surface structure was investigated by scanning electron microscopy (SEM) (Nova Nano SEM). The microsphere diameter was measured using Image-J software, and about 130 microspheres were randomly selected for analysis.

The content of DBZ in microspheres was analyzed by a precipitation method. 5 mg of DBZ-loaded PLGA microspheres were completely dissolved in chloroform. Once dissolved, the chloroform solution was added dropwise into 4 mL of methanol in a centrifuge tube to dissolve DBZ and precipitate PLGA. DBZ in the supernatant was collected and concentrated under nitrogen stream. Then, DBZ was dried in the vacuum oven for overnight and dissolved in 10 mL of 2:1 acetonitrile to millipore water. The product was filtered for high-performance liquid chromatography (HPLC) analysis (Thermo HPLC). The samples were analyzed using a mobile phase of acetonitrile to 0.1% phosphoric acid (50:50) at a flow rate of 1 mL/min on a pentafluorophenylpropyl column and UV detection at 232 nm. Each sample was measured in duplicates. Actual drug loading and drug encapsulation efficiency were calculated using the following equations:

$$Theoretical\ DBZ\ loading\ \% = \frac{DBZ\ (Total)}{DBZ\ (Total) + PLGA} \times 100\%$$
$$(1\text{-}1)$$

$$Actual\ DBZ\ loading\ \% = \frac{DBZ\ (Experiment)}{DBZ\ (Total) + PLGA} \times 100\%$$
$$(1\text{-}2)$$

$$Encapsulation\ efficiency = \frac{Actual\ DBZ\ loading}{Theoretical\ DBZ\ loading} \times 100\%$$
$$(1\text{-}3)$$

*In vitro FITC-dextran release from microspheres*

FITC-dextran released from microspheres was measured by suspending approximately 20 mg microspheres in 5 mL PBS-buffer at pH 7.4. The samples were placed in a shaker maintained at 37 °C and shaken at 200 rpm. At predetermined time intervals, the samples were removed from the shaker and centrifuged at 1000 g for 2 min. 2 mL medium was aliquoted for analysis and fresh medium of equal volume was added thereafter. The precipitated microsphere pellets were resuspended in the medium and placed back in the shaker. *In vitro* release of FITC-dextran was studied in triplicate. The FITC-dextran concentration in the aqueous phase was determined fluorometrically (Excitation: 485 nm, Emission: 520 nm, Synergy H1 microplate reader) using a standard calibration curve.

*In vitro DBZ release from microspheres*

The DBZ-loaded PLGA microspheres were characterized for drug release for 6 days in PBS at pH 7.4 and 37°C. DBZ release was measured using HPLC. In brief, 20 mg samples were placed in individual centrifuge tubes and filled with 2.5 mL of PBS. The samples were then placed in a shaker maintained at 37°C and shaken at 200 rpm. At specific time points, 1 mL medium was removed, saved for analysis, and replaced with same amount of fresh PBS. Time points were chosen such that perfect sink conditions were maintained. Each supernatant sample was extracted with 1 mL chloroform. The organic layer was then separated and allowed to evaporate. The dried DBZ was then reconstituted with 1 mL of a 40% solution of acetonitrile in water to provide a suitable solution for HPLC analysis (Thermo HPLC).

*Isolation of primary preadipocytes*

Primary preadipocytes were collected from limb subcutaneous WAT depots and minced into 2-5 mm$^2$ pieces. Then, these pieces were subject to 1.5mg/mL collagenase digestion with agitation at 37°C for 1.5-2 hours. The digestion was terminated with Dulbecco's modified Eagle's medium (DMEM) containing 10% fetal bovine serum (FBS). After that, the floating mature adipocytes were removed and the cell suspension was filtered through 100 $\mu$m mesh, followed by brief centrifugation at 450 g for 5 min. The pellet was resuspended and seeded onto tissue culture plates.

*Co-culture of primary preadipocytes with microspheres*

Primary preadipocytes were cultured in growth medium containing DMEM, 20% FBS, and 1% penicillin/streptomycin at 37°C with 5% $CO_2$. The medium was changed every other day. Upon confluence, cells were subject to induction medium containing DMEM, 10% FBS, 2.85 $\mu$M insulin, 0.3 $\mu$M dexamethasone, and 0.63 mM 3-isobutyl-methylxanthine for 4 days, followed by differentiation medium containing DMEM, 200 nM insulin, and 10 nM triiodothyronine for 4 more days. Meanwhile, 5 mg polymer microspheres were added in permeable transwell inserts suspended in each well of 24-well plates while cells were induced for adipogenic differentiation. Other parallel treatment groups included cells in DBZ-conditioned medium (10 $\mu$M) and cells in DMSO vehicle control. To monitor adipogenic differentiation, the lipid droplets and nuclei of adipocytes during culture will be counterstained with BODIPY (Red C12) and DAPI, respectively.

*Quantitative polymerase chain reaction*

Total RNA was extracted from cell culture through Trizol. Random hexamer primers were utilized for the reverse transcription to synthesize cDNA. The quantitative polymerase chain reaction (qPCR) was performed with a Roche Light Cycler 96 machine (Roche). The 18S rRNA was applied as an internal control for normalization. For qPCR result analysis, the $2^{-\Delta\Delta ct}$ method was used to calculate the fold change.

*Protein extraction and western blots analysis*

Total protein was extracted from cells or tissue samples using RIPA buffer containing 50 mM Tris-HCl (pH 8.0), 150 mM NaCl, 1% Nonidet P-40, 0.5% sodium deoxycholate, and 0.1% sodium dodecyl sulfate (SDS). Protein concentrations were measured by using Pierce BCA protein assay reagent (Pierce Biotechnology, Rockford, IL, USA). Proteins were separated by SDS-polyacrylamide gel electrophoresis (SDS-PAGE) and then transferred to polyvinylidene fluoride (PVDF) membranes (Millipore Corp., Billerica, MA). Membranes were blocked in 5% milk for 1 hour and incubated with primary antibodies at 4°C overnight. The PGC1-$\alpha$ (sc-13067) and GAPDH (sc-32233) antibodies were purchased from Santa Cruz Biotechnology, and both were diluted 1: 1,000. The horseradish peroxidase (HRP)-conjugated secondary antibody (anti-rabbit IgG, 7074S, Cell Signaling) was diluted 1:5,000. Signals were detected with a ChemiDocTM Touch Imaging System (Bio-rad). The lanes were analyzed for densitometry quantification with Bio-Rad Image Lab V5.2.1

*In vivo injection of PLGA microspheres*

Mice were first anesthetized by a ketamine-xylazine cocktail and then either FITC-dextran-loaded PLGA microspheres or DBZ-loaded PLGA microspheres (20 mg/30 g body weight) were injected into the inguinal WAT depot in one side of the body in a 1 mL solution of 0.5% Methocel E4M (wt/vol; Dow Chemical) and 0.1% Tween-80 (wt/vol; Sigma) in water. Similarly, PLGA microspheres were injected into the contralateral inguinal WAT depot. Adipose tissues were harvested after 24 hours and 14 days post injection for the study with FITC-dextran-loaded PLGA microspheres and DBZ-loaded PLGA microspheres, respectively. Oil Red O staining was performed to label the adipocytes for the study with FITC-dextran-loaded PLGA microspheres whereas hematoxylin and eosin (H&E) and immunohistochemistry staining was conducted for the study with DBZ-loaded PLGA microspheres.

*H&E and immunohistochemistry staining*

Adipose tissues were fixed in 10% formalin for 24 hours at room temperature. Then, the tissues were embedded into paraffin and cut into 4 $\mu$m thick slices, deparaffinized, and rehydrated using xylene, ethanol and water by standard methods. Immunohistochemistry was performed on a Dako Autostainer (Dako). Slides were incubated with 3% hydrogen peroxide and 2.5% normal horse serum (S-2012, Vector), followed by incubation with rabbit polyclonal anti-Ucp1 primary antibody diluted 1:200 in 2.5% normal horse serum (S-2012, Vector) for 60 min. Signals were detected with an anti-rabbit IgG Polymer Detection Kit (MP-7401, Vector). Labeling was visualized with 3,3'-diaminobenzidine (DAB) as the chromogen (SK-4105, Vector). Slides were counterstained with Harris hematoxylin (EK Industries), and whole-slide digital images were collected with an Aperio Scan Scope slide scanner (Aperio).*Statistical analysis*
Quantitative data were reported as mean $\pm$ s.e.m. P-values were calculated by a two-tailed Student's t-test. P<0.05 was considered to be statistically significant.

**Results:**

*Formulation and characterization of PLGA microspheres*

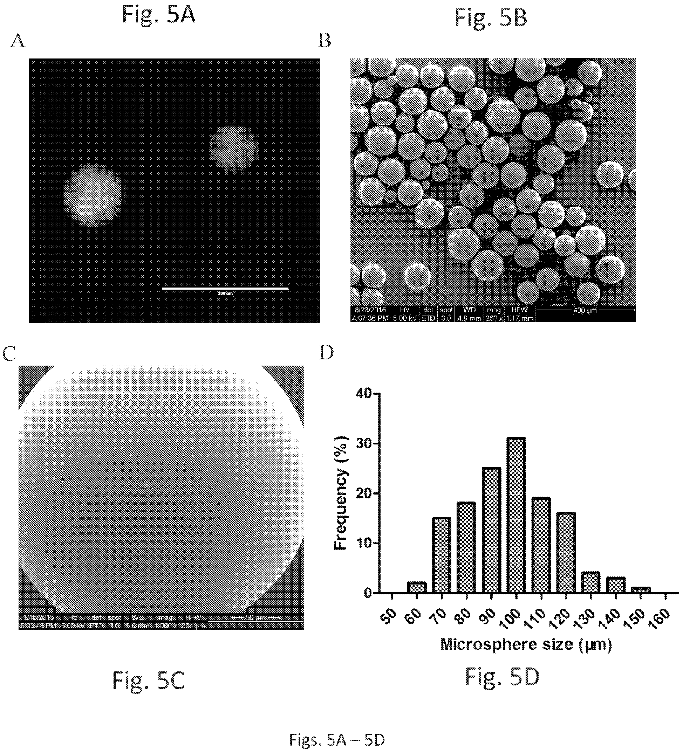

Using PLGA 50:50, drug-loaded PLGA microspheres were prepared and characterized for drug distribution and morphological characteristics. We first encapsulated FITC-dextran, a commonly used fluorescent probe, into PLGA microspheres to assess the distribution of FITC-dextran in microspheres by fluorescent microscopy. It was observed that green fluorescence was uniformly distributed throughout the microspheres (Fig. 5A). Next, we formulated DBZ-loaded PLGA microspheres by optimizing the process parameters such as PVA concentration and stirring speed to obtain a narrow microsphere size range of 50-150 $\mu$m (Fig. 5B). Higher magnification SEM micrograph revealed the smooth surface morphology of the microsphere (Fig. 5C). The size distribution of the DBZ-loaded PLGA microspheres resembled a Gaussian distribution with more than 50% in the range of 90-110 $\mu$m (Fig. 5D). The actual DBZ loading percentage was characterized to be -1.4% (w/w), equivalent to -68% DBZ encapsulation efficiency.

*PLGA microsphere system enables FITC-dextran and DBZ release*

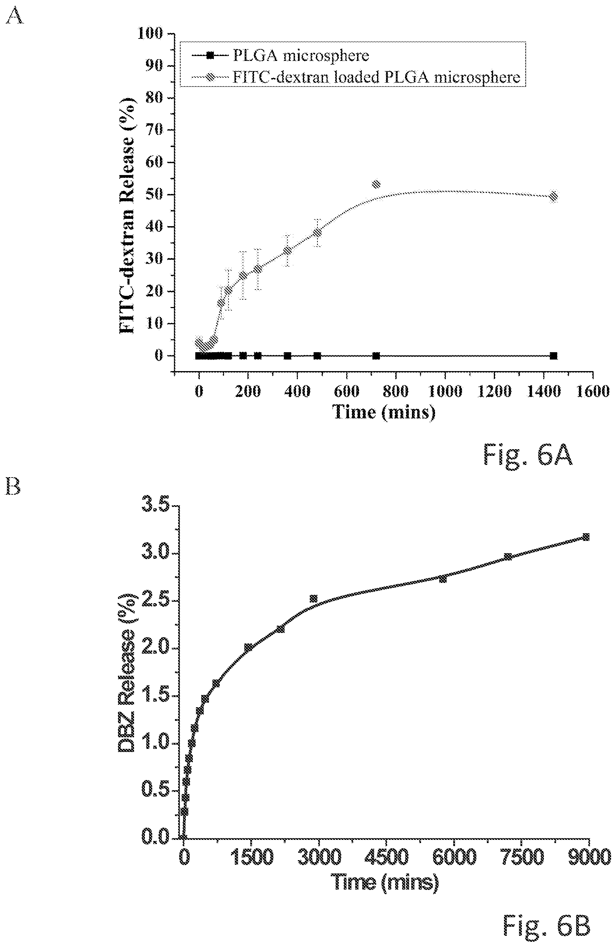

To examine the cargo drug release capabilities from the PLGA microspheres, we first placed the FITC-dextran-loaded PLGA microspheres in the PBS at 37°C and release was quantified fluorometrically. The FITC-dextran-loaded PLGA microspheres showed a rapid release profile, which resulted in about 50% of total FITC-dextran released after 24 hours (Fig. 6A). This is presumably due to the hydrophilic nature of the FITC-dextran. We further characterized the release profile of DBZ from DBZ-loaded PLGA microspheres (Fig. 6B). In general, the release rate of DBZ from PLGA microspheres was much slower than that of FITC-dextran. Approximately, 2% DBZ was released from PLGA microspheres in the first 24 hours. And the release rate became significantly decreased thereafter and around 3% DBZ (~8 $\mu$g) was released over 6 days, indicating that DBZ can be released from PLGA in a sustained manner.

*DBZ-loaded PLGA microspheres promote browning in adipocytes*

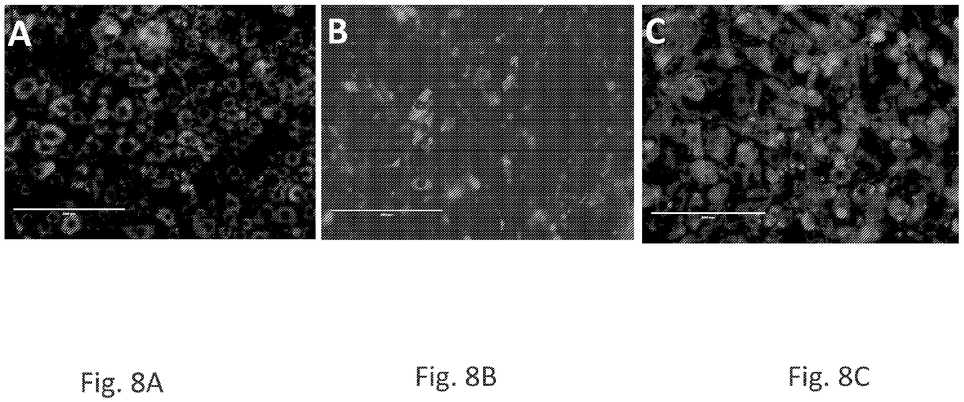

As the initial attempt to test the bioactivity of DBZ released from PLGA microspheres, we co-cultured DBZ-loaded PLGA microspheres with primary adipocytes and examined the cellular responses (Fig. 7A). Following induction of adipogenic differentiation for 8 days, we observed classical lipid droplets characterized by the BODIPY immunofluorescence (Fig. 7A), which suggested that microspheres did not cause obvious adverse effects on cell culture. Then we continued to analyze the mRNA levels of Notch target and thermogenic genes. DBZ-loaded PLGA microspheres significantly inhibited Notch, demonstrated by the 40% mRNA reduction of Notch target genes, *Hes1* and *HeyL* (Fig. 7B). Importantly, DBZ-loaded PLGA microspheres dramatically increased mRNA levels of browning markers, including *Ucp1, Cidea* and *Ppargc1a* (Fig. 7C), as well as mitochondria genes, including *Cox5b, Cox7a, Cpt1a* and *Cpt2* (Fig. 7D), suggesting that DBZ released from PLGA microspheres can inhibit the Notch pathway and consequently promote browning. In addition, western blot results demonstrated that DBZ-loaded PLGA microspheres significantly elevated the protein level of Pgc1-$\alpha$ (encoded by Ppargc1a) by around 3.5 folds (Figs. 7E and 7F), which plays key roles in mitochondrial biogenesis and oxidative metabolism. After 8 days of cell culture, a significant increase was observed in the mitochondrial density of adipocytes treated with DBZ as compared to the control group treated with DMSO (Figs. 8A - 8C). There was an overlap of Red C12 with MitoTracker-positive structures, indicating the potential coordinated enhancement of fatty acid uptake and mitochondria after DBZ treatment. Of potential importance, DBZ-loaded microspheres also resulted in more mitochondria and an overlap of Red C12 with MitoTracker in adipocytes. Collectively, all these data confirmed that DBZ remained bioactive to induce browning after being released from PLGA microspheres *in vitro.*

*DBZ-loaded PLGA microspheres stimulate browning in vivo*

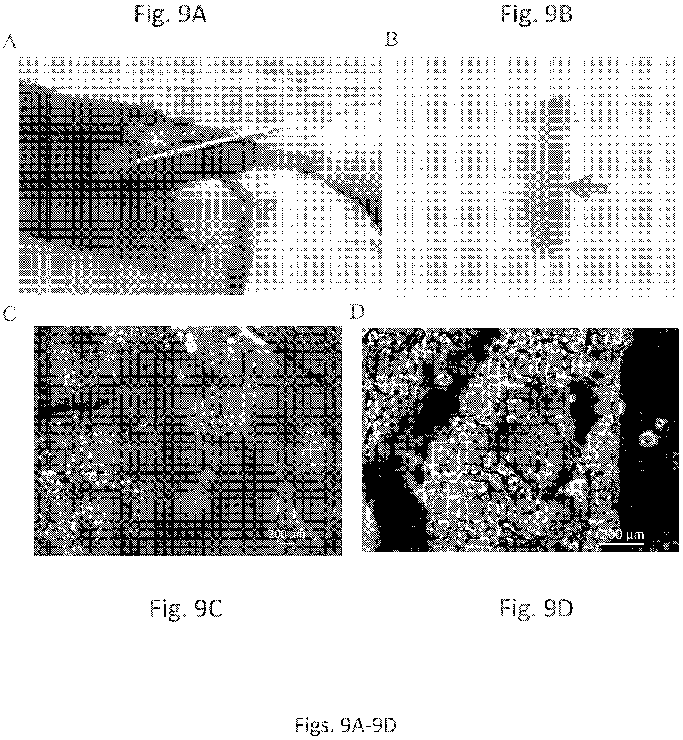

To further test whether PLGA microspheres can locally deliver DBZ and promote browning *in vivo,* we sought to directly inject microspheres to the mouse inguinal WAT depots and assess the browning effect thereafter (Fig. 9A). Initially, to ensure that microspheres could be precisely injected into the depots, we utilized the FITC-dextran-loaded microspheres to track microsphere placement. The microsphere injection sites were readily identified with green fluorescence via gross observation in the target inguinal adipose tissues (Fig. 9B). Following 24 hours of injection, FITC-dextran-loaded microspheres maintained round morphologies with expanded fluorescence, indicating the release of FITC-dextran from the microspheres over this time period (Fig. 9C, D). This observation was also in agreement with our previous results regarding the release profile of FITC-dextran *in vitro.*

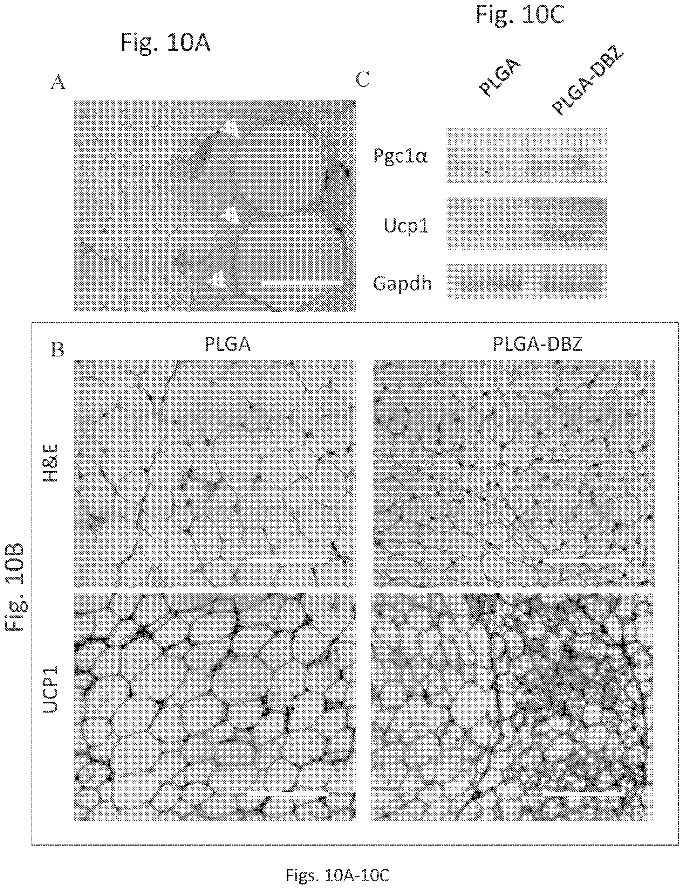

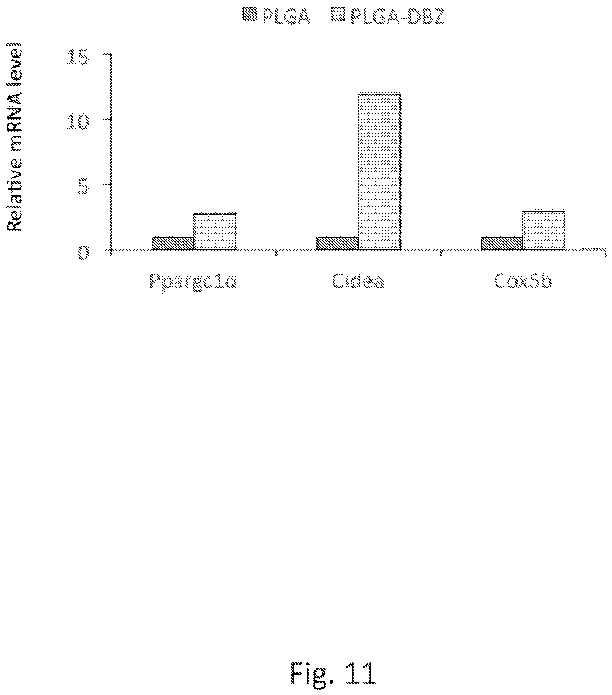

We next injected DBZ-loaded PLGA microspheres into the inguinal WAT depots using the same procedure. After 14 days of injection, the inguinal WAT was collected and processed for paraffin embedding and sectioning. Through H&E staining, we observed that DBZ-loaded PLGA microspheres were dispersed within the WAT and maintained round morphologies in the size range of 50-150 $\mu$m. Importantly, these microspheres did not elicit any significant inflammatory responses (Fig. 10A). Of note, DBZ-loaded PLGA microspheres drastically decreased the sizes of mature white adipocytes (Figure 10B), a hallmark indicator of browning effect. Multilocular Ucp1+beige adipocytes were evidently abundant with DBZ-loaded PLGA microspheres (Figure. 10B). Also, this morphological feature of browning was further supported by western blot of tissue samples exhibiting increased protein levels of Pgc1-$\alpha$ and Ucp1 in the DBZ-loaded microsphere group compared to control (Figure 10C). In addition, mice injected with DBZ-loaded microspheres manifested increased expression of mitochondrial genes (*Cox5b*) as well as brown-fat specific genes such as *Ppargc1a* and *Cidea* relative to controls (Fig. 11). All these data demonstrated that DBZ was successfully released from microspheres and retained its biological activity of Notch inhibition to promote browning *in vivo.*

There is accumulating evidence that significant metabolic differences that distinguish beige adipocytes from white adipocytes can potentially be successfully exploited to establish new therapeutic strategies for treatment and/or prevention of obesity. Thus, identifying and targeting mechanisms underlying browning is crucial in the development of effective therapeutics to reduce adiposity. The Notch signaling pathway is important for cell-cell communication and cell-fate determination during development and is required for adult tissue homeostasis. Notch target gene Hairy/enhancer-of-split 1 (Hes1) inhibits the transcriptions of PR domain-containing 16 (Prdm16), peroxisome proliferator-activated receptor gamma (Ppary) coactivator 1 alpha (Ppargc1a) and Ppary, all critical in the brown adipocyte biogenesis. This leads to reduced mitochondrion numbers and expression of Ucp1. Notably, inhibition of Notch signaling through intraperitoneal injection of DBZ has been found to induce browning of white adipocytes and consequently reduced obesity and improved glucose balance in obese mice. This strategy may open up another novel avenue to treat obesity and its associated metabolic diseases. However, potential compliance issues associated with a requirement for multiple periodic drug injections, in combination with safety and efficacy concerns over widespread drug distribution in the body reduce the translational potential of this treatment. For instance, Notch inhibition has been proposed to affect multiple biological processes, such as angiogenesis, bone formation, and myogenesis. Therefore, it would be beneficial if a carrier system were capable of delivering DBZ locally to the WAT in a sustained manner for treatment of obesity.

Polymer-based controlled drug delivery systems offer several unique advantages over conventional drug delivery including continuous drug release, decreased systemic side effects, and increased patient compliance. Polymer degradation is highly desirable in controlled drug delivery because it eliminates the need for the surgical removal of implants. PLGA is known to degrade by simple hydrolysis of the ester bonds into lactic and glycolic acids, which are ultimately metabolized to carbon dioxide and water. PLGA microspheres have been extensively investigated as carriers to deliver a variety of therapeutic agents. The release of an encapsulated agent from PLGA microspheres is controlled by both diffusion and polymer degradation. The drug release kinetics can be tailored by varying the copolymer composition, molecular weight, and microsphere size. In particular, the degradation rate of PLGA depends on the copolymer composition. The molecular weight loss during hydrolysis is accelerated with an increase in glycolide content. This is attributed to greater absorption of water into the polymer matrix. For example, PLGA exhibited a degradation time in the order of PLGA 50:50 (1-2 months) < PLGA 75:25 (4-5 months) < PLGA 85:15 (5-6 months). Polymers with higher molecular weight result in slower degradation rates. Furthermore, the size of PLGA microspheres is an important controlling factor on drug diffusion and polymer degradation. Berchane *et al.* demonstrated that the initial burst release rate of piroxicam from PLGA microspheres decreased with an increase in microsphere size from 13.9 to 81.2 $\mu$m, which is consistent with Fick's law of diffusion because an increase of diffusion pathways would reduce the drug release rate. It has been reported that large microspheres degrade in a heterogeneous manner wherein the degradation rate in the core is greater than that at the surface. In contrast, microspheres with diameter less than 300 $\mu$m undergo a homogeneous degradation whereby the core degradation rate is equivalent to the surface one. Small microspheres also degrade slower than large microspheres resulting from a decreased accumulation of acidic degradation products. *In vitro* release studies showed that the fabricated DBZ-loaded PLGA microspheres in the size range of 50-150 $\mu$m enabled sustained release of DBZ resulting in ~3% of encapsulated drug released over 6 days. This gradual release will be particularly beneficial to circumvent the need for multiple periodic injections of DBZ for induction of browning.

Co-culture of primary preadipocytes with DBZ-loaded PLGA microspheres demonstrated that released DBZ retained its bioactivity and effectively inhibited Notch signaling and upregulated expression of brown fat specific genes (Fig. 7). For example, the expression of Notch downstream targets *Hes1* and *HeyL* in cultured white adipocytes with both DBZ and DBZ-loaded microspheres were reduced by more than 40%, indicating the bioactivity of DBZ released from polymers. Furthermore, DBZ-loaded microspheres resulted in an elevated expression of browning markers, including *Ucp1, Cidea* and *Ppargc1a,* as well as mitochondria genes, including *Cox5b, Cox7a, Cpt1a* and *Cpt2.* Notably, brown and beige adipocytes contain more mitochondria than white adipocytes, and possess ability to burn lipids (through $\beta$-oxidation) to generate heat. Pgc1-$\alpha$ (encoded by *Ppargc1a*) is also known to induce the expression of Ucp1 and other thermogenic components in adipocytes. Our results are also in line with our earlier findings with the use of DBZ on browning of white adipocytes. The more pronounced increase in *Ucp1* and *Cidea* mRNA levels in the PLGA-DBZ treated groups compared to the DBZ alone groups is presumably due to the continued release of DBZ from microspheres, which should have improved the biological activity of DBZ and thus highlights a major advantage of our drug delivery system for inductive browning.

One of the most important requirements for developing effective polymer-based drug delivery systems is tissue compatibility. PLGA polymers are considered a standard for drug delivery due to their recognized biocompatibility and approval by the Food and Drug Administration for a number of clinical applications. The DBZ-loaded PLGA microspheres were well tolerated following *in vivo* injection into the WAT depots (Fig. 10A). More importantly, we demonstrated that local DBZ-loaded microsphere injection successfully induced browning of WAT after 14 days (Figs. 10B and 10C). The resultant browning further validates that Notch signaling plays a role in regulating the plasticity of white and beige adipocytes *in vivo.*

WAT is the primary site of long-term energy storage. In response to excess calorie intake, the size of the WAT expands through hyperplasia and hypertrophy of adipocytes. Our work pinpointed the potential of polymeric microspheres as a carrier platform to locally deliver DBZ to WAT in a sustained manner. Such a localized delivery system may eliminate the need for repeated injections as well as potential side effects that are accompanied with systemic administration of DBZ. Improved understanding of brown and beige adipocyte biology has led to recent discoveries of a variety of factors to promote WAT browning, such as fibroblast growth factors, bone morphogenetic proteins (BMPs), Irisin, T3 and T4 thyroid hormones, natriuretic peptides, and $\beta$3-adrenergic pathway agonist. However, as with DBZ, there exists significant safety and efficacy concerns on systemic administration of these browning factors as uncontrolled tissue exposure may lead to potential off-target side effects and unpredictable kinetics. For instance, administration of FGF21 is associated with systemic side effects on bone loss. Therefore, development of effective browning-based therapeutics to address the ever-worsening obesity epidemic necessitates the integration of advances in controlled release technologies with discoveries in beige/brown fat cell biology.

DBZ-loaded PLGA microspheres with a size range of 50-150 $\mu$m were prepared as a DBZ delivery system using an emulsion/solvent evaporation technique. The DBZ-loaded PLGA microspheres supported release of DBZ in a sustained manner. The effectiveness of DBZ-loaded PLGA microspheres to induce browning was demonstrated in both *in vitro* and *in vivo* studies. This study for the first time demonstrates the feasibility of developing a bioengineered carrier system for controlled delivery of DBZ to achieve inductive browning using biodegradable polymeric microspheres. To facilitate the translation of our technology platform to have a positive impact in the therapeutic treatment, future efforts will be focused on the evaluation of this controlled delivery system with human cells and tissues.

**EXAMPLE 3: *PPHOSpro-drug system to enable gradual release of $\gamma$-secretase inhibitors:***

PPHOS are high molecular weight polymers with a backbone of alternating phosphorus and nitrogen atoms. Each phosphorus atom bears two organic substituents, with a wide variety of side groups available for property optimization. The type of side group attached to the PPHOS backbone has a profound effect on the physico-chemical and biological properties of the polymer. For example, substitution of chlorine atoms of the poly(dichlorophospazene) with hydrolytically labile groups such as amino acid esters, imidazolyl, glucosyl, glyceryl, and glycolate or lactate ester sensitizes the polymer to break down over periods of hours, days, months, or years at the physiological temperature depending on the specific molecular structure of the polymer. On the other hand, hydrophobic side groups such as aryloxy, fluoroalkoxy and higher alkoxy units protect the polymer backbone against hydrolysis. PPHOS have been investigated for delivering a variety of drugs based on diffusion, erosion, or a mixture of erosion and diffusion. Furthermore, drug molecules can be directly attached as side groups onto polymer chain via hydrolytically sensitive bonds as depots for drug delivery. For example, drug conjugates of biodegradable PPHOS-platinum (II) showed high antitumor activity through the controlled release of the platinum (II) moiety from the PPHOS backbone in the degradation process. Therefore, PPHOS constitute attractive candidate materials for the design of polymer-based pro-drug systems with efficient control over degradation rate and drug release profiles.

**Advantages of PPHOS pro-drug systems include:**

The well-defined polymer structure, which can be reproduced with consistency as the polymers are synthesized via a macromolecular substitution route.

The synthetic flexibility of PPHOS, which allows the development of novel polymers with chemical or biological moieties by exploiting side group chemistry. Co-substitution of $\gamma$-secretase inhibitors with different side groups onto polymer backbone allows for fine-tuning polymer degradation and drug release by simply varying ratio of the substituents.

This polymeric system is biocompatible since the polymer backbone degrades into ammonium phosphates, which can be readily metabolized or excreted by the body.

A reproducible PPHOS-based conjugate, which avoids difficulties that might be encountered by attempting to entrap a drug within a polymer; instead the drug is the polymer. Direct conjugation of DBZ onto polymer backbone help to ensure a high loading efficiency of the drug. Drug release kinetics can be tailored by changing the nature and hydrolytic sensitivity of the polymer-drug linkage.

Such a polymer-drug conjugate system can also be used to develop three-dimensional matrices to either study cell-extracellular microenvironment interactions or generate engineered brown fat for implantation.

***Polymer Synthesis and Characterization:***

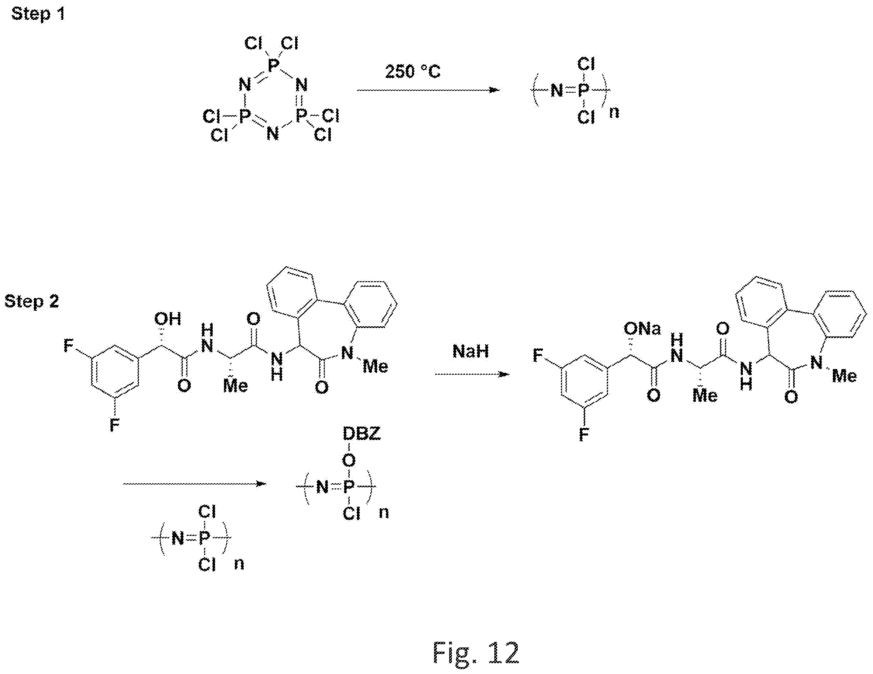

PPHOS-based pro-drug system can be synthesized via a two-step macromolecular substitution protocol. The thermal ring opening polymerization of $(NPCl_2)_3$, followed by nucleophilic substitution reactions of the resultant poly(dichlorophosphazene), results in the production of the PPHOS-$\gamma$-secretase inhibitor pro-drug (Fig. 12). Alternatively, poly(dichlorophosphazene) can be synthesized via the living cationic polymerization of trichlorophosphoranimine. The synthetic details of this approach have been reported in our previous publications. The direct attachment of the sodium salt of a $\gamma$-secretase inhibitor (LY411575) onto the polymer backbone is expected to result in relatively high loading efficiency. The loading percentage and polymer degradation can be further fine-tuned by co-substituting polymer backbone with different side groups. The $^{31}$P NMR (145 MHz) and $^{1}$H NMR (360 MHz) spectra of the polymers will be obtained using a Bruker 360 MHz NMR spectrometer, in order to confirm the structure and side-group ratios. Molecular weights of the polymers will be estimated from results of gel permeation chromatography (GPC) using a Hewlett Packard LC 1100 series.

*Polymer Degradation and Drug Release:*

Polymer degradation is highly desirable for developing controlled/sustained release drug delivery vehicles. The process involved in the polymer degradation generally include several phases: 1) hydration with or without swelling, 2) oligomers and monomers generation by water intrusion, 3) progressive degradation, and 4) oligomers and monomers release leading to mass loss. Developed herein are polymers with a complete degradation time in the range of 12 weeks based on the current intervention protocols to achieve browning of WAT in humans. In brief, PPHOS pro-drug in the form of circular disks (10 mm diameter and 0.5 mm thickness) is incubated in PBS (10 mL) at pH 7.4 at 37°C in a shaker water bath at 250 rpm for 12 weeks. At specific time points, the matrices are removed from the media, dried to constant weight and changes in weight will be determined. The molecular weight of the degraded matrices is also determined using GPC. Samples are visualized by SEM. Analysis of degradation products of these polymers including $\gamma$-secretase inhibitors is performed using a combination of NMR, HPLC, and infrared spectroscopy techniques.

Such a polymeric pro-drug system can be used in different formulations (e.g. microspheres, nanoparticles, fibers, wafers) to deliver $\gamma$-secretase inhibitor to adipose tissues to suppress Notch signaling and induce browning of WAT for treatment of obesity.

**EXAMPLE 4: *Nanoparticle-based delivery system comprising polymersomes:***

Nanoparticulate drug delivery systems comprising polymersomes provide an effective means to improve drug efficacy by providing high drug payload within the target cells. Synthetic block copolymers have been commonly used for the preparation of polymersomes. Polymersomes with a wide range of properties (e.g. different membrane thicknesses, responsiveness to stimuli and permeabilities) can be produced by using block copolymers with different molecular weights, functionalities, charge state, chemical compositions, hydrophilic/hydrophobic ratio, number of blocks and molecular architectures.

*FITC-BSA-loaded Polymersome Formulation and Characterization*

Polystyrene-b-poly(4-vinyl-N-methylpyridine iodide)$_2$, PS-b-(P4MVP)$_2$ triblock polymer was synthesized according to our previous report. FITC-BSA-loaded PS$_{33}$-b-(P4MVP$_{29}$)$_2$ polymersome nanoparticles were prepared via simple dialysis. Specifically, 10 mg of PS$_{33}$-b-(P4MVP$_{29}$)$_2$ triblock copolymer and varying amounts of FITC-BSA were dissolved in DMSO and dialyzed against DI water using Spectrum MWCO 10-12 kDa dialysis tubes to prepare polymersomes at defined concentrations (~5 mg/mL). Free proteins were removed by dialysis (MWCO 100 kDa) for 24 hours. The FITC-BSA concentration in the aqueous phase was determined fluorometrically (Excitation: 485 nm, Emission: 520 nm, Synergy H1 microplate reader) using a standard calibration curve. The actual FITC-BSA loading percentage was characterized to be -8.6% (w/w), equivalent to -86% FITC-BSA encapsulation efficiency.

*Cell Uptake of Polymersome Nanoparticles:*

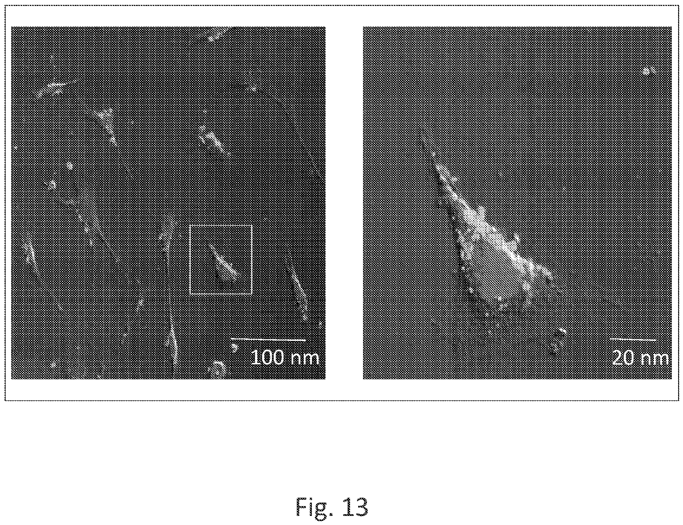

Primary preadipocytes were cultured in growth medium containing DMEM, 20%FBS, and 1% penicillin/streptomycin at 37°C with 5% CO2. The cells were pre-incubated with a series of FITC-BSA-loaded PS$_{33}$-b-(P4MVP$_{29}$)$_2$ polymersome concentrations for 20 min and then incubated with adipocyte medium. After 24 hours, the cells were washed three times with PBS and fixed in 4% formaldehyde. The nuclei were stained with DAPI. Confocal microscopy was performed on a Nikon A1R (Fig. 13).

*DBZ-loaded Polymersome Formulation and Characterization:*

Hydrogenated polybutadiene-b-poly(4-vinyl-N-methylpyridine iodide)$_2$ , HPBD-b-(P4MVP)$_2$ triblock polymer was synthesized via RAFT polymerization. DBZ-loaded *HPBD41-b-(P4MVP$_{25}$)$_2$* polymersome nanoparticles were prepared via simple dialysis. Specifically, 10 mg of *HPBD41-b-(P4MVP$_{25}$)$_2$* triblock copolymer and varying amounts of DBZ were dissolved in DMSO and dialyzed against DI water using Spectrum MWCO 10-12 kDa dialysis tubes for 48 hours to prepare polymersomes at defined concentrations. DBZ-loaded *HPBD41-b-(P4MVP$_{25}$)$_2$* polymersomes were lyophilized to obtain DBZ-loaded *HPBD41-b-(P4MVP$_{25}$)$_2$* nanoparticles.

To test the loading efficiency, 5 mg of *DBZ-loaded HPBD41-b-(P4MP$_{25}$)$_2$* nanoparticles were completely dissolved in 0.4 mL DMSO. The solution was added dropwise into 6 mL of ethyl acetate in a centrifuge tube to dissolve DBZ and precipitate *HPBD41-b-(P4MVP$_{25}$)$_2$*. DBZ in the supernatant was collected and concentrated under nitrogen stream. Then, DBZ was dried in the vacuum oven for overnight and dissolved in 2.4 mL of 2:1 acetonitrile to millipore water. The product was filtered for high-performance liquid chromatography (HPLC) analysis (Thermo HPLC). The samples were analyzed using a mobile phase of acetonitrile to 0.1% phosphoric acid (50:50) at a flow rate of 1 mL/min on a pentafluorophenylpropyl column and UV detection at 232 nm. The DBZ encapsulation efficiency was characterized to be -30%.

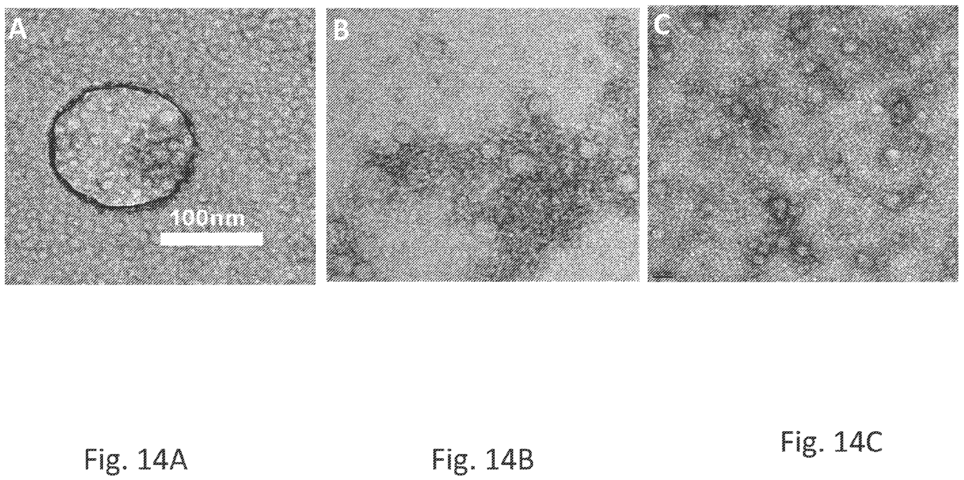

TEM studies were conducted to characterize the size of polymersome nanoparticles (Fig. 14). In brief, DBZ-loaded *HPBD41-b-(P4MVP$_{25}$)$_2$* polymersomes (~2 μL) were put on a 400 mesh ultrathin Type-A grid (Ted Pella) and imaged with Tecnai T20 TEM. An accelerating voltage of 200 kV was used, and the samples were stained with 2% uranyl acetate (UAc) to enhance contrast.

Active targeting strategy can also be developed to improve the delivery efficiency through decorating the nanoparticles with specific ligands targeting specific receptors on adipocytes.

**EXAMPLE 5** *Notch inhibitor-releasing PLGA nanoparticles induce browning of local white adipose tissue to counteract obesity*

In this example, the development of a poly(lactic-co-glycolic acid) (PLGA) nanoparticle (NP) system that can enable selective sustained release of a Notch inhibitor (dibenzazepine, DBZ) to WAT depots is disclosed. These DBZ-NPs inhibit Notch signaling and induce browning after rapid cellular internalization. Importantly, local injection of DBZ-NPs into the inguinal WAT depots in a diet-induced obese mouse model demonstrates localized NP retention, stimulates browning, consequently improves the glucose homeostasis and attenuates body weight gain as compared to the control group. These findings offer new avenues to develop a potential therapeutic strategy for clinical treatment of obesity and its associated metabolic syndrome

**Materials and Methods:**

*Materials:*

The 50:50 Poly(D,L-lactide-co-glycolide) (PLGA, Resomer 504) was obtained from Boehringer Ingelheim (Ingelheim, Germany). The dibenzazepine (DBZ) was purchased from Tocris Bioscience (Bristol, UK). The PLGA with a terminal amine group (PLGA-NH$_2$) was purchased from PolySciTech (West Lafayette, IN). Acetone (Certified ACS) and chloroform were purchased from Fisher Scientific. All other reagents and solvents were purchased from Sigma-Aldrich.

*Animals:*

All procedures regarding animal maintenance and experimental use were performed according to the regulation presented by Purdue University's Animal Care and Use Committee. 10-week-old male die-induced obese (DIO) mice with the C57BL/6 background were purchased from Taconic and housed under normal condition at Purdue University. High-fat diet (Research Diets #D12492, 60% fat) was used to induce and maintain obesity in the mice. Mice with average body weight of 36g at 14 weeks started to receive nanoparticle treatment. After 5 weeks, mice were euthanized by a lethal dose of carbon dioxide, followed by immediate blood collection from hearts for the assessment of serological parameters and then quick dissection of inguinal WAT, BAT and livers for the molecular and cellular analysis.

**Nanoparticle synthesis and characterization.** The PLGA nanoparticles were prepared with a nanoprecipitation method. In brief, PLGA was dissolved in acetone with or without corresponding drug compounds, including DBZ or Nile Red (Fluka Analytical). The polymer/drug mixture solution centrifugation (12,000 rpm, 4 °C, 45 min) and dispersed in water by stirring 15 min. The size and zeta potential were determined by dynamic light scattering (DLS) using Malvern Zetasizer. Nanoparticles were stained with 1% phosphotungstic acid solution and observed for size and morphology using a transmission electron microscope (Philips CM100) operating at 200 kV. The DBZ content in the nanoparticles was determined by HPLC (Thermo Fisher).

**DBZ release from nanoparticles.** The release of DBZ from nanoparticles was examined through dialysis, followed by the HPLC characterization. In detail, 1ml DBZ-NP solution (1mg/ml) was transferred in a dialysis bag (10 KDa) and immersed in 40 ml PBS (pH 7.4) or acetate buffer (pH 5.0) at 37 °C in an orbital shaker at 100 rpm. At specific time points, 12 ml of buffer solution were removed and replaced with fresh. The sample was extracted with chloroform, followed by reconstitution with 1 mL of a 67% solution of acetonitrile in water for HPLC analysis.

**Synthesis of PLGA-Cy5.5 NPs.** The conjugation of a Cy5.5-NHS ester and PLGA-NH$_2$ (M.W. 30,000) was performed by mixing 1.2 mg of Cy5.5-NHS ester and 33 mg of PLGA-NH$_2$ in 3 ml of DMSO in a Schlenk flask under vacuum and stirring overnight at room temperature in darkness. After reaction, the final product was purified by using the solvent/non-solvent technique. The reaction mixture was dropped into 40 ml of methanol cooled in ice bath and centrifuged at 8,000 rpm at 4 °C for 30 min. The pellet was dissolved again in DMSO and the washing procedure was repeated for two more cycles. The final pellet was dried using vacuum oven for 24 hours at room temperature. The PLGA-Cy5.5 NPs were prepared following the standard protocol described above.

**Isolation and culture of the stromal vascular fraction (SVF) from inguinal WAT.** Inguinal WAT was specifically dissected out and minced into small pieces, followed by the digestion of 1.5mg/ml collagenase in Dulbecco's modified Eagle's medium (DMEM) with gentle agitation at 37°C for 1.5-2 h. The digestion was terminated by 10% (vol/vol) fetal bovine serum (FBS) in DMEM. Then the mixture solution was filtered with 100pm cell strainer (BD Flacon) and subjected to a brief centrifugation at 450 g for 5 min. After removal of the floating lipid layer and supernatant, the pellet was resuspended and maintained with DMEM supplemented with 20% (vol/vol) FBS and 1% penicillin/streptomycin at 37°C with 5% $CO_2$.

**Cellular uptake of nanoparticles.** SVF cells were seeded in an 8-well chamber slide (Lab-Tek) at a density of 10,000 cells per well and cultured for 24 h. Then cells were exposed to DMEM containing 0.1mg/ml nile-red loaded PLGA nanoparticles for 10 and 60 min. After washing twice with PBS, cells were subsequently incubated with 50nM LysoTracker Green (Life Technologies) for 30 min and 1mg/ml Hoechst 33342 (Life Technologeis) for 10 min at 37°C. Images were taken with a Leica DMI 6000B fluorescence microscope.

**Adipogenic differentiation of SVF cells with nanoparticles.** SVF cells were cultured in tissue culture plates until confluence. Before adipogenic induction, cells were rinsed with PBS for 3 times and incubated with DMEM containing nanoparticles with the final concentration of 0.1mg/ml for 1 h. After removal of the nanoparticle suspension, cells was rinsed with PBS carefully and subjected to the adipogenic induction medium containing DMEM, 10% (vol/vol) FBS, 2.85 $\mu$M insulin, 0.3 $\mu$M dexamethasone, and 0.63 mM 3-isobutylmethylxanthine for 4 days. At day 5, cells were treated with nanoparticles again with the same procedure described above, followed by the adipogenic differentiation medium containing DMEM, 200 nM insulin, and 10 nM triiodothyronine for 4 more days until adipocytes matured.

**Oxygen consumption rate (OCR) measurement.** SVF cells from inguinal WAT of three different mice were cultured and seeded into the seahorse XF24 microplate (Seahorse Biosciences) at the density of 20,000 cells per well. Cells were induced and differentiated to mature adipocytes with the treatment of PLGA-NP and DBZ-NP following the protocols described above. After 8 days differentiation, mature adipocytes were washed twice with PBS and pre-incubated in XF Base Medium supplemented with 20 mM glucose, 2 mM glutamine and 1 mM pyruvate for 1-2 h at 37 °C in a $CO_2$-free environment. OCR was measured on the XF Extracellular Flux Analyser (Seahorse Biosciences) after sequential injection of Oligomycin (1 mM), FCCP (1 mM), and Rotenone/Antimycin A (0.5 $\mu$M). OCR values presented were normalized to protein mass.

***In vivo* imaging studies.** After anesthetization by a ketamine-xylazine cocktail, mice were injected with 1mg/ml Cy5.5-conjugated PLGA nanoparticles suspended in PBS in the inguinal WAT. Images were taken with the IVIS Lumina II imaging system (Caliper, USA) at 24,48 and 72 h post injection. After the 72 h scanning, mice were euthanized and multiple related tissues were collected, followed by the *ex vivo* imaging. The fluorescent images were analyzed by the Living Image Software.

**Measurements of blood glucose.** For GTT, mice were intraperitoneally injected with 100 mg/ml D-glucose (2 g kg-1 body weight) after 16 h fasting. Blood was drawn from tails and glucose levels were measured by a glucometer (Accu-Check Active, Roche). For ITT, mice were intraperitoneally injected with human insulin (Santa Cruz) (0.75 U per kg body weight) after 4 h fasting. Glucose concentrations were measured in the blood drawn from tails. For both GTT and ITT, mice were separated into one per cage with random orders.

**Measurement of serological parameters.** Serum levels of free fatty acids, glucose, cholesterol, and triglycerides from fasted DIO mice after treatment of nanoparticles for 5 weeks were examined by standard kits from Randox Laboratories. The insulin level in the serum was measured by ELISA according to the manufacturer's instructions (ALPCO).

**H&E and immunohistochemistry staining.** Portions of inguinal WAT were fixed in 10% formalin for 24 h at room temperature, embedded into paraffin and cut into 4-$\mu$m slides. Slides were deparaffinized and incubated with xylene, ethanol and water sequentially for rehydration according to standard protocols. For H&E staining, slides were stained with haematoxylin for 15 min, followed by eosin staining for 1 min. For immunohitstochemistry staining, slides were incubated in 0.01 M sodium citrate (pH 6.0) and heated at 96 °C for 20 min to retrieve antigens. Ucp1 Immunohistochemistry was conducted in a Dako Autostainer (Dako, Carpinteria, CA). Slides were stained with the Ucp1 primary antibody (ab10983, Abcam) at 1:200 dilution for 1h. For signal detection, the anti-rabbit IgG Polymer Detection Kit (MP-7401, Vector) was utilized. Then 3,3'-diaminobenzidine (DAB) was used as the chromogen to visualize the labelling. Images were collected through an Aperio Scan Scope slide scanner (Aperio, Vista, CA).

**Oil red O staining.** After cryosection, liver samples were stained with the Oil red O working solution containing 60% (vol/vol) Oil red O stock solution (5 g 1-1 in isopropanol) for 30 min. After staining, the slides were washed with 60% isopropanol and images were taken.

**Total RNA extraction, cDNA synthesis and quantitative PCR.** Total RNA was extracted from cells or tissues through Trizol (Life Technologies) in accordance with the manufacture's protocol. After the measurement of RNA concentration by the Nanodrop (Thermo Fisher), 5$\mu$g RNA was reversed transcribed to cDNA with random hexamer primers by M-MLV reverse transcriptase (Invitrogen). The quantitative PCR was performed on the Roche Light Cycler 96 PCR System (Roche). The sequences of primers were listed in the supplemental table S For the result analysis, the $2^{-\Delta\Delta ct}$ method was used to calculate the relevant changes of gene expression after normalization to the 18S rRNA.

**Protein extraction and western blot.** Protein was extracted from cell or tissue samples with RIPA buffer containing 50 mM Tris-HCl (pH 8.0), 150 mM NaCl, 1% Nonidet P-40, 0.5% sodium deoxycholate, and 0.1% sodium dodecyl sulfate (SDS). Protein concentration was determined by Pierce BCA protein assay reagent (Pierce Biotechnology, Rockford, IL, USA). Proteins were separated by SDS-PAGE and then transferred to PVDF membranes (Millipore Corp., Billerica, MA), followed by the blocking with 5% milk for 1 h. Then the membranes were incubated with primary antibodies at 4°C overnight. The Hes1 (SC-166378), Pgc1-$\alpha$ (SC-13067) and $\beta$-actin (SC-47778) antibodies (Santa Cruz Biotechnology) were all diluted 1:1,000. The HRP-conjugated secondary antibodies (anti-rabbit IgG, 7074S or anti-mouse IgG, 7076S, Cell Signaling) were diluted 1:5,000. After incubation with secondary antibodies for 2 h, the enhanced chemiluminescence western blotting substrate (Pierce Biotechnology) was used to detect signals with a ChemiDocTM Touch Imaging System (Bio-rad).

**Statistical analysis.** Quantitative data were represented as mean $\pm$ s.e.m. P-values were calculated by a two-tailed Student's t-test. P<0.05 was considered to be statistically significant.

**Results:**

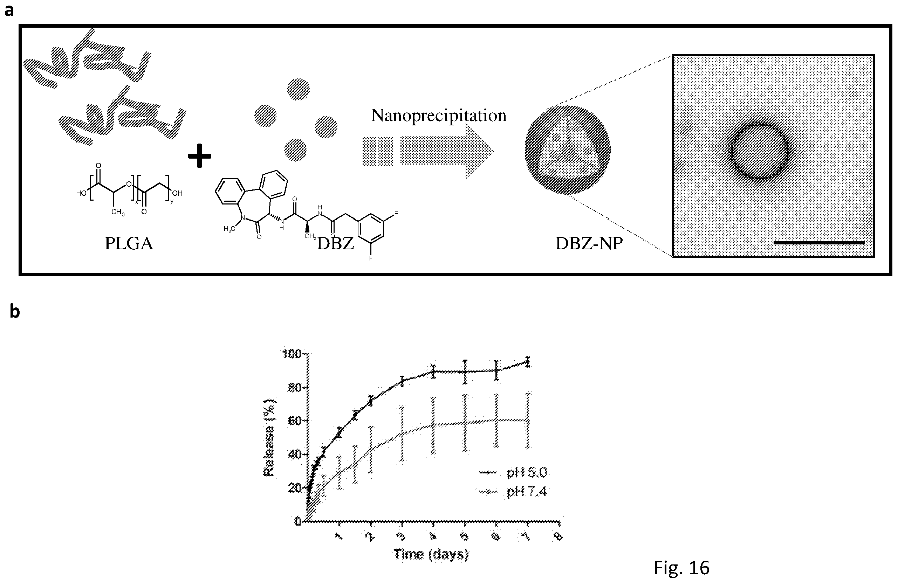

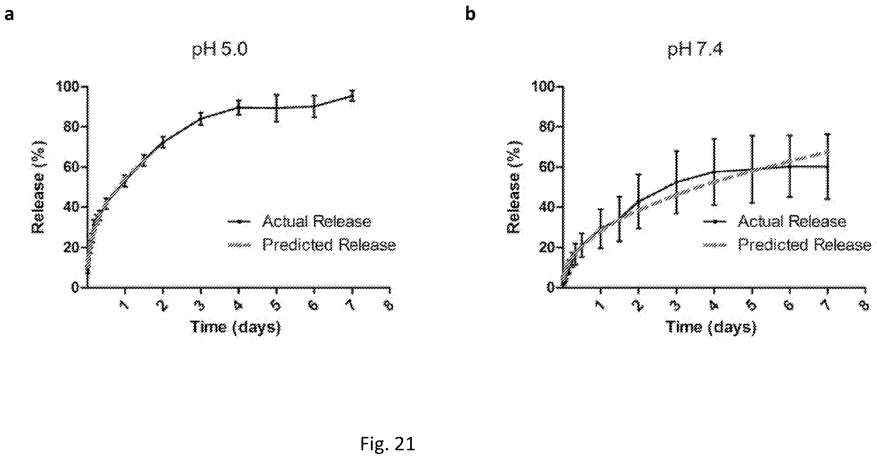

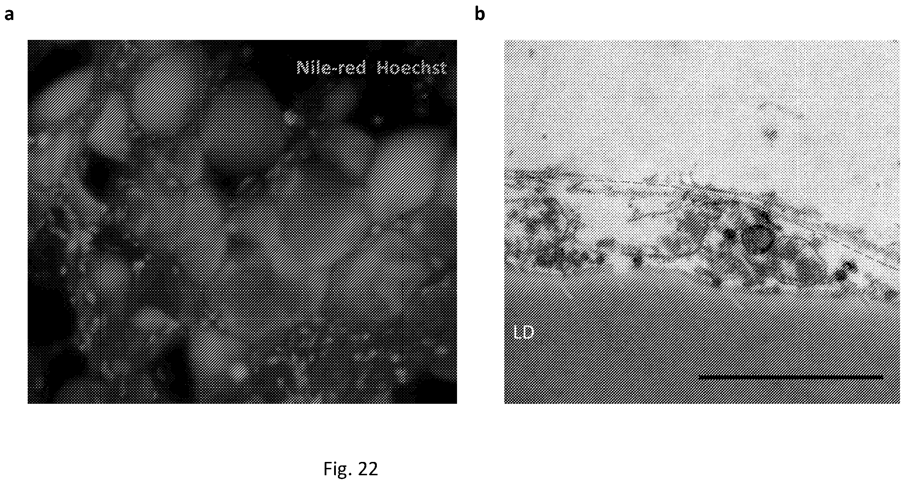

**Synthesis and characterization of nanoparticles.** The DBZ-NPs were formulated at a DBZ/PLGA mass ratio of 1:19 using a nanoprecipitation method with optimized conditions (Tab. 1), which resulted in a high encapsulation efficiency of 94%. Transmission electron microscopy (TEM) showed the spherical structures of synthesized DBZ-NPs (Fig. 16a). As shown in Fig. 16b, the nanoparticle size was determined to be ~150 nm by the dynamic light scattering (DLS). Similarly sized bare PLGA-NPs were used as controls. The surface zeta potential for the DBZ-NPs in water was -20.2 $\pm$ 3.0 mV. To examine the stability of the resulting nanoparticles, they were incubated in buffered saline solutions at different pH values and then monitored by DLS for the particle size and the polydispersity index (PDI) (Fig. 21). No significant differences in the particle size and PDI were found at different pH conditions over 7 days. To evaluate DBZ release characteristics, we investigate *in vitro* release profiles of DBZ from DBZ-NPs under neutral (1 $\times$ PBS buffer, pH 7.4) and acidic (1 $\times$ PBS buffer, pH 5.0) conditions at 37°C. The DBZ-NPs presented continuous release of DBZ with a half-life of 1 day at pH 5.0 and 3 days at pH 7.4, respectively (Fig. 16c). In addition, the DBZ release kinetics from these DBZ-NPs were further analyzed by mathematical modelling (Tab. 2). The release data fitted well to the Korsmeyer-Peppas model at both pH values (Fig. 22), indicating that DBZ is released by diffusion.

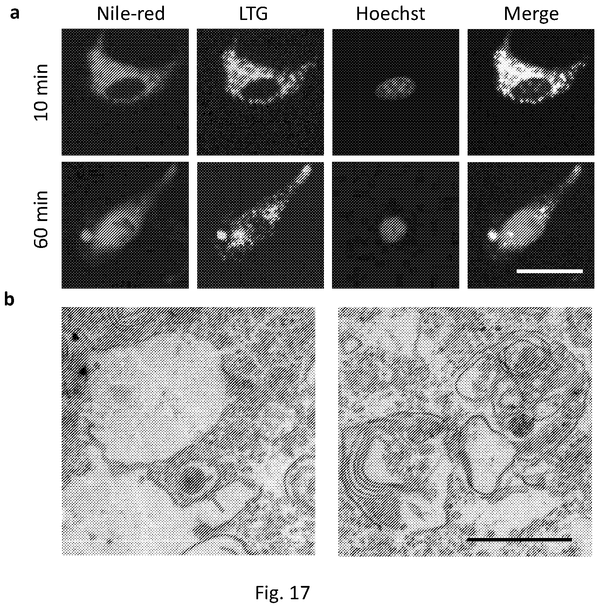

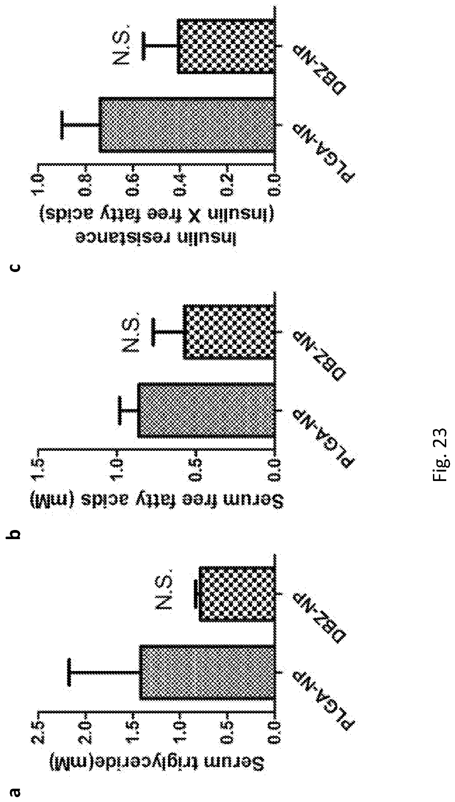

**Cellular uptake of nanoparticles.** The cellular uptake of NPs by primary preadipocytes was examined using the confocal laser scanning microscopy (Fig. 17a). NPs were visualized by encapsulating a nile red fluorescent dye while the late endo/lysosomes were stained with LysoTracker Green (LTG) . The stromal vascular fractions (SVF) were isolated from inguinal WAT and cultured, followed by the incubation with nanoparticles. Within 10 min, the late endo/lysosomes were colocalized with NPs in pre-adipocytes as evident from the appearance of orange to yellow fluorescence, indicating a relatively rapid endocytosis of NPs. When the incubation time was prolonged to 60 min, NPs were distributed throughout the cytoplasm and around the nucleus suggesting the endolysosomal escape of NPs. Next, the endocytosis process of NPs was further visualized by TEM imaging (Fig. 17b). After 10 min of incubation, NPs were clearly identified in both the endocytic vesicles and the endosomes, in agreement with the results discussed above. To further validate the cellular uptake of NPs at the tissue level, we retrieved inguinal WAT from mice and subsequently incubated with nile red-loaded NPs . After 24 h of incubation, the red fluorescence was observed in a host of mature adipocytes (Fig. 23a). The cellular uptake of nanoparticles was further confirmed by the TEM imaging of inguinal WAT (Fig. 23b), demonstrating the localization of nanoparticles within the adipocytes. Our data suggested that the NPs were rapidly internalized by adipocytes through the endocytotic pathway.

**DBZ-NPs inhibit Notch and promote browning effects *in vitro*.** To test the bioactivity of our DBZ-NPs in suppressing Notch signaling and stimulate browning, we induced adipogenic differentiation of SVF cells together with the treatment of DBZ-NPs or control PLGA NPs. After 8 days of differentiation, we examined the mRNA levels of the Notch targets and the browning markers by quantitative PCR assay. The classical Notch target genes, *Hey1, HeyL,* and *Hes1,* were significantly decreased with the treatment of the DBZ-NPs (Fig. 18a). Meanwhile, the mRNA levels of the browning markers, including *Ucpl, Cidea, Ppargcla* and *Dio2,* were elevated dramatically (Fig. 18b). Provided that browning effects are intrinsically associated with the mitochondria biogenesis and oxidative capacity, the mRNA level of the cytochrome C oxidative subunits 5b (*Cox5b*) was increased significantly by the treatment of the DBZ-NPs (by 69%, Fig. 18c). Consistently, the protein level of Hes1 was decreased while that of PGC1$\alpha$ was increased markedly, which was quantified by the densitometry analysis (Fig. 18d), suggesting the inhibition of Notch and the browning effect by the DBZ-NPs. In addition, in order to determine whether these observed molecular alterations can be reflected by the cellular respiration, the oxygen consumption rate (OCR) of the differentiated adipocytes was investigated in an XF24 analyzer. With the DBZ-NP treatment, OCR under both basal and proton leak conditions were significantly increased (Fig. 18e). Collectively, these results suggested that DBZ released from NPs retained its bioactivity to inhibit Notch and thus induce browning *in vitro.*

**The local retention of DBZ-NPs leads to the browning of inguinal WAT *in vivo*.** To assess the local NP retention capability within the WAT, the *in vivo* biodistribution of Cy5.5-labelled PLGA NPs (Cy5.5-NPs) following injection into the inguinal WAT depots was investigated using a non-invasive near-infrared optical imaging technique. The near infrared fluorescence dye Cy5.5-NHS ester was conjugated to PLGA-NH$_2$ through amide bond formation, followed by the standard formulation of nanoparticles. Those nanoparticles were subsequently injected into the inguinal WAT depots, which constitute the widely investigated fat tissue due to its significant capability to recruit beige adipocytes. After 24 h of administration, Cy5.5-NPs presented an intensive fluorescence signal around the injection site. As time increased, the fluorescence intensity of Cy5.5-NPs decreased with fluorescence signals retained in the WAT depot until 72 h post injection, indicating a robust NP retention effect within the WAT (Fig. 19a). After 72 h of injection, the mice were immediately euthanized and relevant tissues including inguinal WAT (iWAT), anterior subcutaneous WAT (asWAT), BAT, muscle, liver, spleen, kidney, and heart were harvested for *ex vivo* imaging. It is noteworthy that the fluorescence signal of Cy5.5-NPs was exclusively detected in the inguinal WAT while absent in the off-target tissues (Fig. 19b), which was in agreement with the findings on the local NP retention within iWAT. In addition, TEM analysis of iWAT further confirmed the localization of nanoparticles within the adipocytes after *in vivo* injection (Fig. 19c). Altogether, these data supported the potential of nanoparticles in achieving local intracellular delivery to the inguinal WAT depots while minimizing off-target effects. We next evaluated the efficacy of our DBZ-NPs in inhibiting Notch and consequently promoting browning *in vivo*. We employed the diet induced obese (DIO) mouse model and injected DBZ-NPs to the inguinal WAT depots once per week for 5 weeks. After that, total RNA was extracted from inguinal WAT, followed by the quantitative PCR analysis. The mRNA level of *UCP1* was upregulated sixfold in the DBZ-NP group compared to the control PLGA-NP group (Fig. 19d). Consistently, the mRNA levels of mitochondria genes *Cox5b* and *Cox7a* were increased dramatically by the DBZ-NPs compared to the PLGA-NPs (Fig. 19e). These molecular changes were corroborated by the H&E and UCP1 immunohistochemical staining of the inguinal WAT, demonstrating multilocular lipid droplets, shrinkage of adipocytes, and elevated UCP1 expression (Fig. 19f), a hallmark indicator of browning effect resulting from the DBZ-NP treatment. Thus, local delivery of DBZ-NPs could effectively inhibit Notch signaling and induce browning of local WAT *in vivo*.

**DBZ-NPs improve metabolism and prevent diet-induced obesity.** We further sought to assess whether the inductive browning of the local inguinal WAT depots by DBZ-NPs could regulate metabolism and reduce obesity in obese mice. We used the high-fat diet (HFD) induced obese model and injected DBZ-NPs to the inguinal WAT depots once per week to determine the effects of the DBZ-NPs on body weight, adipocyte tissue morphology, and glucose metabolism. There was no difference in body weight gain between the mice treated with Rosi (in solution and nontargeted NP forms) and nontreated obese mice. Notably, the DBZ-NP treatment attenuated the body-weight gain seen in the PLGA-NP-treated control group without affecting energy intake (Fig. 20a, b). After 5 weeks of treatment, mice were euthanized and tissue samples were retrieved. The inguinal WAT with the DBZ-NP treatment appeared smaller in size compared to the PLGA-NP counterparts, which was further verified by the lower weight of the inguinal WAT from DBZ-NP treated mice (Fig. 20c). More strikingly, the local browning effects by the DBZ-NPs led to significantly enhanced glucose tolerance (Fig. 20d) and insulin sensitivity (Fig. 20e). Consistently, plasma chemistry analysis demonstrated a significant 54% reduction of glucose level in the mice treated with DBZ-NPs than those with PLGA-NPs (Fig. 20f). Furthermore, there was a 47% decrease in blood cholesterol level in mice receiving DBZ-NPs (Fig. 20g). No statistical differences were observed in levels of plasma insulin (Fig. 20h), triglyceride, free fatty acids and insulin resistance (IR) (Fig. 23) in mice treated with DBZ-NPs and PLGA-NPs. These results together provide encouraging evidence that local delivery of DBZ-NPs to WAT can exert significant positive metabolic effects in prevention of obesity in a DIO mouse model.

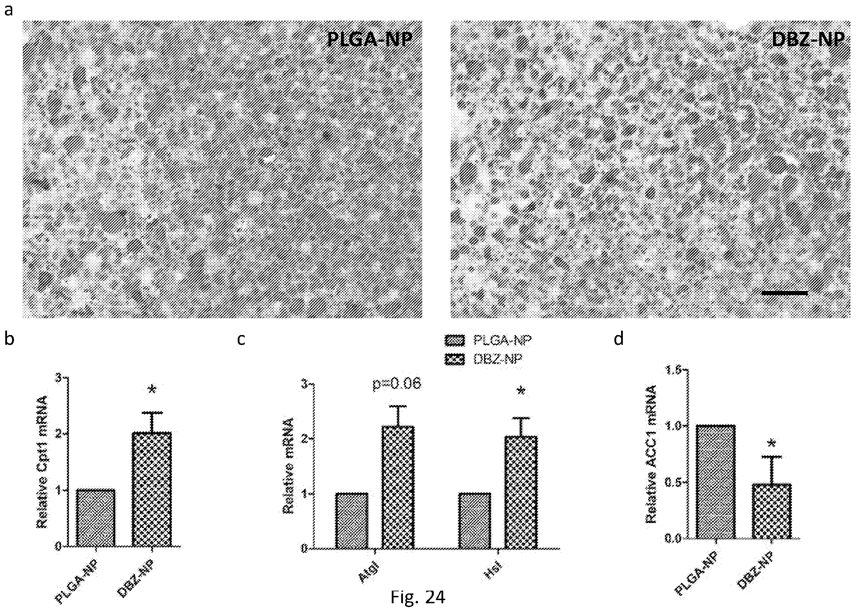

In addition, the lipid content was reduced in the liver of mice treated with DBZ-NPs than in the mice treated with PLGA-NPs (Fig. 24a). This was further confirmed by the qPCR analysis of genes involved in hepatic fatty acid metabolism. The mRNA level of carnitine palmitoyltransferase I (*Cpt1*) was significantly increased by the DBZ-NP treatment (Fig. 24b), indicating an elevated fatty acid oxidation. And the mRNA levels of adipose triglyceride lipase (*Atgl*) and hormone-sensitive lipase (*Hsl*), both of which contribute to lipolysis in the liver, were profoundly increased by the DBZ-NP treatment (Fig. 24c). Meanwhile, the *de novo* lipogenesis was significantly reduced in the liver of mice receiving DBZ-NPs, manifested by the decreased mRNA level of acetyl-CoA carboxylase 1 (*ACC1*) (Fig. 24d), the rate-limiting step in fatty acid synthesis. Collectively, these data suggested that the local browning by DBZ-NPs enhanced hepatic lipid metabolism under the HFD-fed conditions.

These systemic benefits elicited by the local manipulation are also in agreement with the previous publication that the transplantation of either subcutaneous WAT from exercise-trained mice or BAT from normal mice significantly improved glucose tolerance and insulin sensitivity.

A plethora of browning factors have been discovered to improve energy expenditure over the past few years, such as β3-adrenergic pathway agonists[27, 28], BMPs[29, 30], FGFs[31, 32, 33], Irisin[34], thyroid hormones[35, 36], and natriuretic peptides[37]. However, these factors have been facing significant translational challenges due to uncontrolled tissue exposure and unpredictable kinetics with systemic administration. For example, although FGF21 has been a promising drug for type 2 diabetes due to its powerful capability to modulate the glucose and lipid metabolism, systemic administration of FGF21 is associated with the skeletal fragility[38]. Therefore, there is a critical need to develop an efficient drug delivery platform to advance the clinical translation. The engineered polymeric nanoparticles disclosed herein could be potentially applied to deliver a broad spectrum of these browning factors. Moreover, it may be also advantageous to incorporate different browning factors simultaneously to achieve the synergistic effects.

In this example, we designed and synthesized a polymeric NP platform through nanoprecipitation of biodegradable and biocompatible PLGA for direct intracellular delivery of DBZ to WAT depots. These DBZ-loaded PLGA nanoparticles (DBZ-NPs) were optimized for particle size and surface properties, and were rapidly internalized by adipocytes during *in vitro* cell culture. Furthermore, we showed that DBZ released from these DBZ-NPs resulted in inhibition of Notch target genes and the elevation of browning marker and mitochondria genes of adipocytes. Importantly, local injection of these engineered DBZ-NPs into the inguinal WAT depots in a diet-induced obese mouse model demonstrated localized NP retention in the depot without any accumulation in other major organs, stimulated browning of WA, consequently improved the glucose homeostasis, and attenuated body weight gain as compared to the control group. These findings not only provide mechanistic insights into the direct contribution of the local browning of WAT induced by the Notch inhibition to the systemic metabolic improvements but also offer new avenues to develop a potential therapeutic strategy based on browning of WAT depots for clinical treatment of obesity and its associated metabolic syndrome.

Table 1

| Factors | Optimized values |
|---|---|
| PLGA concentration | 0.02 g/mL |
| Acetone Volume | 5 mL |
| PVA concentration | 0.5% |
| Stirring speed | 600 rpm |
| Temperature | 20 °C |
| $H_2O$ Volume | 100 mL |

Table 2

| Model | | pH 5.0 | | pH 7.4 | |
|---|---|---|---|---|---|
| | | Average | s.e.m | Average | s.e.m |
| Zero-Order $Q_t = Q_0 + K_0t$ | K (%*min-1) | 0.013 | 0.001 | 0.008 | 0.002 |
| | Readjusted | 0.277 | 0.099 | 0.668 | 0.076 |
| | AIC | 170.338 | 2.213 | 141.432 | 12.451 |
| | MSC | 0.231 | 0.131 | 1.043 | 0.223 |
| First-Order $\ln Q_t = \ln Q_0 + K_1t$ | K (%*min-1) | 0.001 | 0.000 | 0.000 | 0.000 |
| | Readjusted | 0.859 | 0.031 | 0.848 | 0.076 |
| | AIC | 140.113 | 2.917 | 118.756 | 10.070 |
| | MSC | 1.910 | 0.259 | 2.303 | 0.877 |
| Higuchi model $Q_t=K_H(t)^{1/2}$ | K (%*min-1/2) | 1.517 | 0.087 | 0.695 | 0.193 |
| | Readjusted | 0.911 | 0.031 | 0.947 | 0.008 |
| | AIC | 67.788 | 4.281 | 108.736 | 10.730 |
| | MSC | 2.365 | 0.322 | 2.859 | 0.151 |
| Korsmeyer-Peppas Model $Q_t/Q_\infty=K_k t^n$ | K (%*min-n) | 3.514 | 0.584 | 1.184 | 0.465 |
| | n | 0.380 | 0.017 | 0.455 | 0.039 |
| | Readjusted | 0.988 | 0.003 | 0.959 | 0.005 |
| | AIC | 44.558 | 4.696 | 105.300 | 10.621 |
| | MSC | 4.301 | 0.296 | 3.050 | 0.130 |

Table 3

| Gene | Sequences | SEQ ID NO |
|---|---|---|
| Hey1 | 5'-TGA ATC CAG ATG ACC AGC TAC TGT-3' | 1 |
| | 5'-TAC TTT CAGACT CCG ATC GCT TAC-3' | 2 |
| HeyL | 5'-CAG ATG CAA GCC CGG AAG AA-3' | 3 |
| | 5'-ACC AGA GGC ATG GAG CAT CT-3' | 4 |
| Hes1 | 5'-GCA CAG AAA GTC ATC AAA GCC-3' | 5 |
| | 5'-TTG ATC TGG GTA ATG CAG TTG-3' | 6 |
| UCP1 | 5'-AGG CTT CCA GTA CCA TTA GGT-3' | 7 |
| | 5'-CTG AGT GAG GCA AAG CTG ATT T-3' | 8 |
| Ppargc1a | 5'-AGC CGT GAC CAC TGA CAA CGA G-3' | 9 |
| | 5'-GCT GCA TGG TTC TGA GTG CTA AG-3' | 10 |
| Cidea | 5'-TGA CAT TCA TGG GAT TGC AGA C-3' | 11 |
| | 5'-GGC CAG TTG TGA TGA CTA AGA C-3' | 12 |
| Dio2 | 5'-AAT TAT GCC TCG GAG AAG ACC G-3' | 13 |
| | 5'-GGC AGT TGC CTA GTG AAA GGT-3' | 14 |
| Cox5b | 5'-TTC AAG GTT ACT TCG CGG AGT-3' | 15 |
| | 5'-CGG GAC TAG ATT AGG GTC TTC C-3' | 16 |
| Cox7a | 5'-GCT CTG GTC CGG TCT TTT AGC-3' | 17 |
| | 5'-GTA CTG GGA GGT CAT TGT CGG-3' | 18 |

**Claims**

1. A therapeutic agent for use in the treatment of a disease which benefits from selectively inducing browning of adipocytes in a subject comprising:

   at least one compound or composition that induces the formation of brown and/or beige adipocytes, wherein the at least one compound or composition is an inhibitor to the Notch signaling pathway, wherein said inhibitor is capable of upregulating the expression of uncoupling protein-1 (*Ucp1*) or *Ppargcla;*
   a polymer that forms microparticles or nanoparticles and that encapsulates the at least one compound or composition; and
   wherein the therapeutic agent is injected into white adipose tissue depots (WAT).

2. The therapeutic agent for use according to claim 1, wherein said inhibitor to Notch signaling pathway is selected from γ-secretase inhibitors including dibenzazepine (DBZ) and (N-[N-(3,5-difluorophenacetyl)-1-alanyl]-S-phenylglycine t-butyl ester) (DAPT).

3. The therapeutic agent for use according to claim 1, wherein the polymer

   - is selected from the group consisting of synthetic polymers and natural polymers, and/or
   - is selected from the group consisting of biodegradable polymers and non-degradable polymers; and/or
   - comprises a class of artificial vesicles made from synthetic amphiphilic block copolymers, wherein said artificial vesicles comprise hollow spheres that contain an aqueous solution in the core surrounded by a bi-layer membrane, and/or
   - the polymer is poly (lactic-co-glycolic acid) (PLGA).

4. A therapeutic agent for use according to claim 1, comprising:

   at least one compound or composition that induces the formation of brown and/or beige adipocytes, wherein the at least one compound or composition is an inhibitor to the Notch signaling pathway, wherein said inhibitor is capable of upregulating the expression of uncoupling protein-1 (*Ucp1*) or *Ppargc1a*; and
   a polymer that forms microparticles or nanoparticles and encapsulates the at least one compound or composition for use in the treatment of obesity in a subject, wherein the therapeutic agent is injected into white adipose tissue depots (WAT).

**5.** A therapeutic agent for use according to claim 1, comprising

at least one compound or composition that induces the formation of brown and/or beige adipocytes, wherein the at least one compound or composition is an inhibitor to the Notch signaling pathway, wherein said inhibitor is capable of upregulating the expression of uncoupling protein-1 (*Ucp1*) or *Ppargc1a*; and
a polymer that forms microparticles or nanoparticles and that encapsulates the at least one compound or composition for use in the treatment of diabetes in a subject, wherein the therapeutic agent is injected into white adipose tissue depots (WAT).

**6.** The therapeutic agent for use according to claim 4 or 5, wherein the encapsulation of the drug within the polymer matrix system is developed by physical interactions selected from any one of or a combination of hydrophobic interaction, hydrophilic interaction, hydrogen bonding, and inter-molecular electrostatic interactions, and/or wherein the encapsulation of the drug within the polymer matrix system is developed through a conjugation linkage between the drug and the polymer via at least one functional group.

**7.** The therapeutic agent for use according to claim 6, wherein the polymer-drug conjugation linkage is formed by any one of or a combination of amine reaction, thiol reactions (e.g thiol click reactions), carboxylate reaction, hydroxyl reactions, aldehyde and ketone reactions, active hydrogen reactions, photo-chemical reaction, and cycloaddition reactions (e.g. Diels-Alder reaction, copper-catalyzed azide-alkyne cycloaddition (CuAAC), copper-free azide-alkyne huisgen cycloaddition).

**8.** The therapeutic agent for use according to claim 6, wherein the at least one functional group is selected from the group comprises any one of or a combination of amines, thiol, carboxylic acid, aldehyde, ketone, active hydrogen sites on aromatic ring, diene, azide isothiocyanates, isocyanates, acyl azides. N-hydroxysuccinimide-(NHS)-ester, sulfo-NHS, sulfonyl chloride, aldehydes, epoxides, carbonates, aryl halide, imidoesters, carbodiimides (e.g. N,N'-dicyclohexylcarbodiimide (DCC) and 1-ethyl-3-(3-dimethylaminopropyl)carbodiimide (EDC), alkylphosphate compound, anhydride, fluorophenyl ester, hydroxymethyl phosphine, guanidino group, iodoacetyl derivative, maleimides, aziridines, acryloyl derivatives, arylating agents, disulfide derivative, vinylsulfone, phenylthioester, cisplatin, diazoacetate, carbonyl diimidazole, oxiranes, N,N'-disuccinimidyl carbonate (DSC), N-hydroxylsuccinimidyl chloroformate, alkyl halogens, hydrazine, maleimide, alkyne, and phosphorus-bound chlorine.

**9.** The therapeutic agent for use according to claim 6, wherein the conjugated linkage comprises any one of or a combination of isothiourea, isourea, amide, sulfonamide, shift-base, secondary amine, carbamate, arylamine, amidine, phosphoramidate, thioether, disulfide, β-thiosulfonyl, ester, carbamate, hydrazone, diazo, 2+4 cycloaddition, 1,2,3-triazoles, carbohydrates, amino acid esters bond.

**10.** The therapeutic agent for use according to claim 4 or 5, wherein the polymer matrix system comprises

- synthetic polymers comprising any one of or a combination of poly (aliphatic ester) (e.g. poly(lactide) (PLA), poly(ε-caprolactone) (PCL), poly(glycolic acid) (PGA), poly(lactic-co-glycolic acid) (PLGA), poly(trimethylene carbonate) (PTMC), polydioxanone (PDS), poly(ortho ester), polyanhydrides, poly(anhydride-coimide), poly(anhydride-esters), polyurethanes(e.g. Degrapols), poly(glycerol sebacate), poly(ethylene imine), poly(acrylic acid)(PAA), polyethylene glycol (PEG), poly(vinyl alcohol) (PVA), poly(N-isopropylacrylamide) (PNIPAm), poly(oxazolines) (e.g. poly(2-methyloxazoline and poly(2-ethyl-2-oxazoline), oligo(ethylene glycol) fumarates (OPFs), polypropylene fumarate), poly(alkyl cyanoacrylates), polyacrylic amide, synthetic poly(amino acids) (e.g. poly(L-glutamic acid) (L-PGA) and poly(aspartic acid), polyphosphazenes, and poly(phosphoesters) and blends thereof, and/or
- natural polymers comprising any one of or a combination of fibrin, collagen, matrigel, elastin, elastin-like peptides, albumin, natural poly(amino acids) (e.g. cyanophycin, poly(ε-L-lysine) and poly(γ-glutamic acid)), and polysaccharides (e.g. hyaluronic acid, chitosan, dextran, chondroitin sulfate, agarose, alginate, and heparin), and blends thereof, and/or
- non-degradable polymers.

**11.** The therapeutic agent for use according to claim 10, wherein non-biodegradable polymers comprise any one of or a combination of poly(ethyl ethylene) (PEE), poly(butadiene) (PBD), poly(dimethylsiloxane) (PDMS), and poly(styrene) (PS), poly(N-ethyl-4-vinypyridinium), poly(2,2-(dimethyl aminoethyl methacrylate), poly(ethylene imine), poly(allylamine), and poly(diallyl dimethyl ammonium chloride), poly(acrylic acid), poly(styrene sulfonate), poly(vinyl sulfate), and poly(3-sulfopropyl methacrylate), and polyacrylic amide and blends thereof.

**12.** The therapeutic agent for use according to any one of claims 4-11, wherein the polymer matrix system comprise any one of or a combination of linear, star-shaped, hyper-branched, and crosslinked architectures.

**Patentansprüche**

**1.** Therapeutisches Mittel zur Verwendung bei der Behandlung einer Krankheit, die von der selektiven Induktion der Bräunung von Adipozyten in einem Subjekt profitiert, umfassend:

mindestens eine Verbindung oder Zusammensetzung, die die Bildung von braunen und/oder beigen Adipozyten induziert, wobei die mindestens eine Verbindung oder Zusammensetzung ein Inhibitor des Notch-Signalwegs ist, wobei der Inhibitor die Expression von Entkoppelndem Protein-1 (*Ucp1*) oder *Ppargcla* hochregulieren kann; ein Polymer, das Mikropartikel oder Nanopartikel bildet und die mindestens eine Verbindung oder Zusammensetzung verkapselt; und wobei das therapeutische Mittel in weiße Fettgewebedepots (*white adipose tissue,* WAT) injiziert wird.

**2.** Therapeutisches Mittel zur Verwendung nach Anspruch 1, wobei der Inhibitor des Notch-Signalwegs aus γ-Sekretase-Inhibitoren einschließlich Dibenzazepin (DBZ) und (N-[N-(3,5-Difluorphenacetyl)-1-alanyl]-S-phenylglycin-t-butylester) (DAPT) ausgewählt ist.

**3.** Therapeutisches Mittel zur Verwendung nach Anspruch 1, wobei das Polymer

- ausgewählt ist aus der Gruppe bestehend aus synthetischen Polymeren und natürlichen Polymeren, und/oder
- ausgewählt ist aus der Gruppe der biologisch abbaubaren Polymere und der nicht abbaubaren Polymere; und/oder
- eine Klasse von künstlichen Vesikeln umfasst, die aus synthetischen amphiphilen Blockcopolymeren hergestellt sind, wobei die künstlichen Vesikel Hohlkugeln umfassen, die eine wässrige Lösung im Kern enthalten, der von einer zweischichtigen Membran umgeben ist, und/oder
- das Polymer Poly(lactid-co-glykolsäure) (PLGA) ist.

**4.** Therapeutisches Mittel zur Verwendung nach Anspruch 1, umfassend:

mindestens eine Verbindung oder Zusammensetzung, die die Bildung von braunen und/oder beigen Adipozyten induziert, wobei die mindestens eine Verbindung oder Zusammensetzung ein Inhibitor des Notch-Signalwegs ist, wobei der Inhibitor die Expression von Entkoppelndem Protein-1 *(Ucp1)* oder *Ppargc1a* hochregulieren kann; und ein Polymer, das Mikropartikel oder Nanopartikel bildet und die mindestens eine Verbindung oder Zusammensetzung verkapselt, zur Verwendung bei der Behandlung von Fettleibigkeit in einem Subjekt, wobei das therapeutische Mittel in weiße Fettgewebedepots (WAT) injiziert wird.

**5.** Therapeutisches Mittel zur Verwendung nach Anspruch 1, umfassend

mindestens eine Verbindung oder Zusammensetzung, die die Bildung von braunen und/oder beigen Adipozyten induziert, wobei die mindestens eine Verbindung oder Zusammensetzung ein Inhibitor des Notch-Signalwegs ist, wobei der Inhibitor die Expression von Entkoppelndem Protein-1 *(Ucp1)* oder *Ppargc1a* hochregulieren kann; und ein Polymer, das Mikropartikel oder Nanopartikel bildet und das die mindestens eine Verbindung oder Zusammensetzung verkapselt, zur Verwendung bei der Behandlung von Diabetes in einem Subjekt, wobei das therapeutische Mittel in weiße Fettgewebedepots (WAT) injiziert wird.

**6.** Therapeutisches Mittel zur Verwendung nach Anspruch 4 oder 5, wobei die Verkapselung des Wirkstoffs in dem Polymermatrixsystems gebildet wird durch physikalische Wechselwirkungen ausgewählt aus einer oder einer Kombination von hydrophoben Wechselwirkungen, hydrophilen Wechselwirkungen, Wasserstoffbrückenbindungen und intermolekularen elektrostatischen Wechselwirkungen, und/oder wobei die Verkapselung des Wirkstoffs in dem Polymermatrixsystems gebildet wird durch eine Konjugationsbindung zwischen dem Wirkstoff und dem Polymer über mindestens eine funktionelle Gruppe.

**7.** Therapeutisches Mittel zur Verwendung gemäß Anspruch 6, wobei die Polymer-Wirkstoff-Konjugationsbindung gebildet wird durch eines oder eine Kombination von Aminreaktion, Thiolreaktionen (z. B. Thiol-Klickreaktionen), Carboxylatreaktion, Hydroxylreaktionen, Aldehyd- und Ketonreaktionen, Reaktionen mit aktivem Wasserstoff, photochemischer Reaktion und Cycloadditionsreaktionen (z. B. Diels-Alder-Reaktion, kupferkatalysierter Azid-Alkin-Cycloaddition (CuAAC), kupferfreier Azid-Alkin-Huisgen-Cycloaddition).

8. Therapeutisches Mittel zur Verwendung nach Anspruch 6, wobei die mindestens eine funktionelle Gruppe ausgewählt ist aus der Gruppe umfassend eines oder eine Kombination von Aminen, Thiol, Carbonsäure, Aldehyd, Keton, aktiven Wasserstoffstellen an einem aromatischen Ring, Dien, Azidisothiocyanaten, Isocyanaten, Acylaziden. N-Hydroxysuccinimid-(NHS)-Ester, Sulfo-NHS, Sulfonylchlorid, Aldehyden, Epoxiden, Carbonaten, Arylhalogenid, Imidestern, Carbodiimiden (z. B. N,N'-Dicyclohexylcarbodiimid (DCC) und 1-Ethyl-3-(3-dimethylaminopropyl)carbodiimid (EDC), Alkylphosphatverbindung, Anhydrid, Fluorphenylester, Hydroxymethylphosphin, Guanidingruppe, Jodacetylderivat, Maleimiden, Aziridinen, Acryloylderivaten, Arylierungsmitteln, Disulfidderivaten, Vinylsulfon, Phenylthioester, Cisplatin, Diazoacetat, Carbonyldiimidazol, Oxiranen, N,N'-Disuccinimidylcarbonat (DSC), N-Hydroxylsuccinimidylchlorformat, Alkylhalogenen, Hydrazin, Maleimid, Alkin und phosphorgebundenes Chlor.

9. Therapeutisches Mittel zur Verwendung nach Anspruch 6, wobei die konjugierte Bindung eines oder eine Kombination von Isothioharnstoff, Isoharnstoff, Amid, Sulfonamid, Shift-Base, sekundärem Amin, Carbamat, Arylamin, Amidin, Phosphoramidat, Thioether, Disulfid, β-Thiosulfonyl, Ester, Carbamat, Hydrazon, Diazo, 2+4-Cycloaddition, 1,2,3-Triazolen, Kohlenhydraten, Aminosäureesterbindung umfasst.

10. Therapeutisches Mittel zur Verwendung nach Anspruch 4 oder 5, wobei das Polymermatrixsystem umfasst

   - synthetische Polymere, umfassend eines von oder eine Kombination von Poly(aliphatischem Ester) (z.B. Poly(lactid) (PLA), Poly(ε-caprolacton) (PCL), Poly(glykolsäure) (PGA), Poly(lactid-co-glykolsäure) (PLGA), Poly(trimethylencarbonat) (PTMC), Polydioxanon (PDS), Poly(orthoester), Polyanhydriden, Poly(anhydridcoimid), Poly(anhydridestern), Polyurethanen (z. B. Degrapole), Poly(glycerinsebacat), Poly(ethylenimin), Poly(acrylsäure) (PAA), Polyethylenglykol (PEG), Poly(vinylalkohol) (PVA), Poly(N-isopropylacrylamid) (PNIPAm), Poly(oxazolinen) (z. B. Poly(2-methyloxazolin und Poly(2-ethyl-2-oxazolin), Oligo(ethylenglykol)fumaraten (OPFs), Poly(propylenfumarat), Poly(alkylcyanoacrylaten), Polyacrylamid, synthetische Poly(aminosäuren) (z. B. Poly(L-glutaminsäure) (L-PGA) und Poly(asparaginsäure), Polyphosphazenen und Poly(phosphoestern) und Mischungen davon, und/oder
   - natürliche Polymere, umfassend eines oder eine Kombination von Fibrin, Kollagen, Matrigel, Elastin, elastinähnlichen Peptiden, Albumin, natürlichen Poly(aminosäuren) (z. B. Cyanophycin, Poly(ε-L-Lysin) und Poly(γ-Glutaminsäure)) und Polysacchariden (z. B. Hyaluronsäure, Chitosan, Dextran, Chondroitinsulfat, Agarose, Alginat und Heparin) sowie Mischungen davon und/oder
   - nicht abbaubare Polymere.

11. Therapeutisches Mittel zur Verwendung nach Anspruch 10, wobei nicht biologisch abbaubare Polymere eines oder eine Kombination von Poly(ethylethylen) (PEE), Poly(butadien) (PBD), Poly(dimethylsiloxan) (PDMS) und Poly(styrol) (PS), Poly(N-ethyl-4-vinypyridinium), Poly(2,2-(dimethylaminoethylmethacrylat), Poly(ethylenimin), Poly(allylamin) und Poly(diallyldimethylammoniumchlorid), Poly(acrylsäure), Poly(styrolsulfonat), Poly(vinylsulfat) und Poly(3-sulfopropylmethacrylat) sowie Polyacrylamid und Mischungen davon umfassen.

12. Therapeutisches Mittel zur Verwendung nach einem der Ansprüche 4-11, wobei das Polymermatrixsystem eines oder eine Kombination von linearen, sternförmigen, hyperverzweigten und vernetzten Architekturen umfasst.

**Revendications**

1. Agent thérapeutique destiné à être utilisé dans le traitement d'une maladie qui bénéficie de l'induction sélective du brunissement des adipocytes chez un sujet comprenant :

   au moins un composé ou au moins une composition qui induit la formation d'adipocytes bruns et/ou beiges, ledit au moins un composé ou ladite au moins une composition étant un inhibiteur de la voie de signalisation Notch, ledit inhibiteur étant capable de réguler à la hausse l'expression de la protéine de découplage 1 (Ucp1) ou du Ppargcla ;
   un polymère qui forme des microparticules ou des nanoparticules et qui encapsule ledit au moins un composé ou ladite au moins une composition ; et

ledit agent thérapeutique étant injecté dans des dépôts de tissu adipeux blanc (WAT).

2. Agent thérapeutique destiné à être utilisé selon la revendication 1, ledit inhibiteur de la voie de signalisation Notch étant choisi parmi les inhibiteurs de la γ-sécrétase comprenant la dibenzazépine (DBZ) et le t-butylester de N-[N-(3,5-difluorophénacétyl)-1-alanyl]-S-phénylglycine) (DAPT).

3. Agent thérapeutique destiné à être utilisé selon la revendication 1, ledit polymère

    - étant choisi dans le groupe constitué par les polymères synthétiques et les polymères naturels, et/ou
    - étant choisi dans le groupe constitué par les polymères biodégradables et les polymères non dégradables ; et/ou
    - comprenant une classe de vésicules artificielles fabriquées à partir de copolymères séquencés amphiphiles synthétiques, lesdites vésicules artificielles comprenant des billes creuses qui contiennent une solution aqueuse dans le noyau entouré d'une membrane bicouche, et/ou
    - ledit polymère étant l'acide poly(lactique-co-glycolique) (PLGA).

4. Agent thérapeutique destiné à être utilisé selon la revendication 1, comprenant :

    au moins un composé ou au moins une composition qui induit la formation d'adipocytes bruns et/ou beiges, ledit au moins un composé ou ladite au moins une composition étant un inhibiteur de la voie de signalisation Notch, ledit inhibiteur étant capable de réguler à la hausse l'expression de la protéine de découplage 1 (*Ucp1*) ou du *Ppargc1a* ; et
    un polymère qui forme des microparticules ou des nanoparticules et encapsule ledit au moins un composé ou ladite au moins une composition destiné à être utilisé dans le traitement de l'obésité chez un sujet, ledit agent thérapeutique étant injecté dans des dépôts de tissu adipeux blanc (WAT).

5. Agent thérapeutique destiné à être utilisé selon la revendication 1, comprenant

    au moins un composé ou au moins une composition qui induit la formation d'adipocytes bruns et/ou beiges, ledit au moins un composé ou ladite au moins une composition étant un inhibiteur de la voie de signalisation Notch, ledit inhibiteur étant capable de réguler à la hausse l'expression de la protéine de découplage 1 (*Ucp1*) ou du *Ppargc1a* ; et
    un polymère qui forme des microparticules ou des nanoparticules et qui encapsule ledit au moins un composé ou ladite au moins une composition destiné à être utilisé dans le traitement du diabète chez un sujet, ledit agent thérapeutique étant injecté dans des dépôts de tissu adipeux blanc (WAT).

6. Agent thérapeutique destiné à être utilisé selon la revendication 4 ou 5, ladite encapsulation du médicament à l'intérieur du système de matrice polymère étant développée par des interactions physiques choisies parmi une quelconque interaction parmi, ou une combinaison de, une interaction hydrophobe, une interaction hydrophile, une liaison hydrogène et des interactions électrostatiques intermoléculaires, et/ou ladite encapsulation du médicament à l'intérieur du système de matrice polymère étant développée par une liaison de conjugaison entre le médicament et le polymère par l'intermédiaire d'au moins un groupe fonctionnel.

7. Agent thérapeutique destiné à être utilisé selon la revendication 6, ladite liaison de conjugaison polymère-médicament étant formée par une quelconque réaction parmi, ou une combinaison de, une réaction d'amine, les réactions de thiol (par exemple réactions clic de thiol), une réaction de carboxylate, les réactions d'hydroxy, les réactions d'aldéhyde et de cétone, les réactions d'hydrogène actif, une réaction photochimique et les réactions de cycloaddition (par ex., la réaction de Diels-Alder, une cycloaddition azoture-alcyne catalysée par le cuivre (CuAAC), une cycloaddition azoture-alcyne de Huisgen sans cuivre).

8. Agent thérapeutique destiné à être utilisé selon la revendication 6, ledit au moins un groupe fonctionnel étant choisi dans le groupe comprenant un quelconque élément parmi, ou une combinaison de, les groupes amine, un groupe thiol, un groupe acide carboxylique, un groupe aldéhyde, un groupe cétone, les sites à hydrogène actif sur un cycle aromatique, un groupe diène, les groupes isothiocyanate d'azoture, les groupes isocyanate, les groupes azotures d'acyle, un groupe N-hydroxysuccinimide-(NHS)-ester, un groupe sulfo-NHS, un groupe chlorure de sulfonyle, les groupes aldéhyde, les groupes époxy, les groupes carbonate, un groupe halogénure d'aryle, les groupes imidoester, les groupes carbodiimide (par ex. les groupes N,N'-dicyclohexylcarbodiimide (DCC) et 1-éthyl-3-(3-diméthylaminopropyl)carbodiimide (EDC), un composé alkylphosphate, un groupe anhydride, un groupe ester de fluorophényle, un groupe hydroxyméthylphosphine, un groupe guanidino, un dérivé d'iodoacétyle, les groupes maléimide, les groupes aziridine, les dérivés d'acryloyle, les agents d'arylation, un dérivé de disulfure, un groupe vinylsulfone, un groupe phénylthioester, un groupe cisplatine, un groupe diazoacétate, un groupe diimidazole de carbonyle, les groupes oxirane, un groupe carbonate de N,N'-disuccinimidyle (DSC), un groupe chloroformiate de N-hydroxylsuccinimidyle, les groupes halogène d'alkyle, un groupe hydrazine, un groupe maléimide, un groupe alcyne et un atome de chlore lié à un atome de phosphore.

9. Agent thérapeutique destiné à être utilisé selon la revendication 6, ladite liaison conjuguée comprenant un quelconque élément parmi, ou une combinaison de, une isothiourée, une isourée, un amide, un sulfonamide, une base de décalage, une amine secondaire, un carbamate, une arylamine, une amidine, un phosphoramidate, un thioéther, un disulfure, un $\beta$-thiosulfonyle, un ester, un carbamate, une hydrazone, un diazo, une cycloaddition 2+4, les 1,2,3-triazoles, les glucides, une liaison d'esters d'acides aminés.

10. Agent thérapeutique destiné à être utilisé selon la revendication 4 ou 5, ledit système de matrice polymère comprenant

- des polymères synthétiques comprenant un quelconque polymère ou une combinaison de, un polyester aliphatique (par exemple le polylactide (PLA), la poly-$\epsilon$-caprolactone (PCL), l'acide polyglycolique (PGA), l'acide poly(lactique-co-glycolique) (PLGA), le polycarbonate de triméthylène (PTMC), la polydioxanone (PDS), le polyorthoester, les polyanhydrides, le poly(anhydride-co-imide), les poly(anhydride-esters), les polyuréthanes (par ex. les degrapols), le poly(sébacate de glycérol), la polyéthylène imine, l'acide polyacrylique (PAA), le polyéthylène glycol (PEG), l'alcool polyvinylique (PVA), le poly-N-isopropylacrylamide (PNIPAm), les polyoxazolines (par ex. la poly(2-méthyloxazoline et la poly(2-éthyl-2-oxazoline), les fumarates d'oligo(éthylène glycol) (OPF), le fumarate de polypropylène, les polycyanoacrylates d'alkyle, l'amide polyacrylique, les acides polyaminés synthétiques (par ex. l'acide poly-L-glutamique) (L-PGA) et l'acide polyaspartique, les polyphosphazènes et les polyphosphoesters et leurs mélanges, et/ou
- des polymères naturels comprenant un quelconque polymère parmi, ou une combinaison de, la fibrine, un collagène, le matrigel, l'élastine, les peptides de type élastine, l'albumine, les acides polyaminés naturels (par exemple la cyanophycine, la poly-$\epsilon$-L-lysine et l'acide poly-$\gamma$-glutamique), et les polysaccharides (par ex. l'acide hyaluronique, le chitosane, le dextrane, le sulfate de chondroïtine, l'agarose, l'alginate et l'héparine), et leurs mélanges, et/ou
- des polymères non dégradables.

11. Agent thérapeutique destiné à être utilisé selon la revendication 10, lesdits polymères non biodégradables comprenant un quelconque polymère parmi, ou une combinaison de, le polyéthyléthylène (PEE), le polybutadiène (PBD), le polydiméthylsiloxane (PDMS) et le polystyrène (PS), le poly-N-éthyl-4-vinypyridinium, le poly-2,2-méthacrylate de diméthylaminoéthyle, la polyéthylène imine, la polyallylamine et le polychlorure de diallyldiméthylammonium, l'acide polyacrylique, le sulfonate de polystyrène, le sulfate de polyvinyle et le polyméthacrylate de 3-sulfopropyle, et l'amide polyacrylique et leurs mélanges.

12. Agent thérapeutique destiné à être utilisé selon l'une quelconque des revendications 4 à 11, ledit système de matrice polymère comprenant une quelconque architecture parmi ou une combinaison de, les architectures linéaires, en forme d'étoile, hyper-ramifiées et réticulées.

Fig. 1A

Fig. 1B

## Fig. 2A

## Fig. 2B

Fig. 2C

Fig. 2D

Figs. 2A -2D

Fig. 3A

Fig. 3B

Figs. 3A – 3E

Fig. 4

## Fig. 5A

## Fig. 5B

## Fig. 5C

## Fig. 5D

Figs. 5A – 5D

A

Fig. 6A

B

Fig. 6B

Figs. 7A-7F

Fig. 8A                              Fig. 8B                              Fig. 8C

EP 3 368 553 B1

Fig. 9A

Fig. 9B

A

B

C

D

Fig. 9C

Fig. 9D

Figs. 9A-9D

Fig. 10A

Fig. 10C

A

C

PLGA

PLGA-DBZ

Pgc1α

Ucp1

Gapdh

B          PLGA                    PLGA-DBZ

H&E

UCP1

Fig. 10B

Figs. 10A-10C

Fig. 11

Step 1

Step 2

Fig. 12

Fig. 13

Fig. 14A

Fig. 14B

Fig. 14C

Fig. 15

Fig. 16

Fig. 17

Fig. 18

Fig. 19

Fig. 20

a

b

pH 5.0

pH 7.4

Fig. 21

Fig. 22

Fig. 23

EP 3 368 553 B1

Fig. 24

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62247192
- WO 2015123576 A
- US 2014057837 A
- EP 2085120 A
- US 2015010630 A
- WO 2015120065 A

**Non-patent literature cited in the description**

- **PENGPENG BI et al.** Inhibition of Notch signaling promotes browning of white adipose tissue and ameliorates obesity. *NATURE MEDICINE,* 01 August 2014, vol. 20 (8), 911-918